# EUROPÄISCHE PATENTSCHRIFT

(11) **EP 1 966 111 B1**
(45) Veröffentlichungstag und Bekanntmachung des Hinweises auf die Patenterteilung: **01.02.2012**
(21) Anmeldenummer: 06830823.8
(22) Anmeldetag: 22.12.2006
(51) Int. Cl.: C07C 5/10, C07C 13/18

(54) **VERFAHREN ZUR UMSETZUNG EINES AROMATISCHEN KOHLENWASSERSTOFFS IN GEGENWART VON WASSERSTOFF**
PROCESS FOR REACTING AN AROMATIC HYDROCARBON IN THE PRESENCE OF HYDROGEN
PROCEDE DE TRANSFORMATION D UN HYDROCARBURE AROMATIQUE EN PRESENCE D HYDROGENE

(30) Priorität: 23.12.2005 DE 102005062354
(43) Veröffentlichungstag der Anmeldung: 10.09.2008
(73) Patentinhaber: BASF SE, 67056 Ludwigshafen (DE)
(72) Erfinder: BECKER, Michael, 77654 Offenburg (DE); SALDEN, Axel, 70190 Stuttgart (DE); STAECK, Bianca, 68165 Mannheim (DE); HENKELMANN, Jochen, 68165 Mannheim (DE); SPRINGMANN, Steffen, 70193 Stuttgart (DE); VAN LAAR, Frederik, Dubai (AE); RUPPEL, Wilhelm, 68163 Mannheim (DE); RESCH, Peter, 67310 Hettenleidelheim (DE); BENDER, Michael, 67061 Ludwigshafen (DE)
(86) Internationale Anmeldenummer: PCT/EP2006/070186
(87) Internationale Veröffentlichungsnummer: WO 2007/074147

(56) Entgegenhaltungen:
- GB-A- 1 104 275
- GB-A- 1 157 356
- US-A1- 2003 113 598

## Beschreibung

Die vorliegende Erfindung betrifft ein Verfahren zur Umsetzung eines aromatischen Kohlenwasserstoffes, der schwefelhaltige aromatische Verbindungen enthält, oder eines Gemisches aus aromatischen Kohlenwasserstoffen, das schwefelhaltige aromatische Verbindungen enthält, ggf. in Gegenwart von Wasserstoff, wobei in einem ersten Schritt schwefelhaltige aromatische Verbindungen entfernt werden (Schritt a), und in einem zweiten Schritt der aromatische Kohlenwasserstoff oder das Gemisch der aromatischen Kohlenwasserstoffe in Gegenwart eines geträgerten Rutheniumkatalysators in Gegenwart von Wasserstoff hydriert wird (Schritt b).

In einer Ausführungsform betrifft die vorliegende Erfindung ein Verfahren, wobei der aromatische Kohlenwasserstoff Benzol ist. In einer weiteren Ausführungsform betrifft die vorliegende Erfindung ein Verfahren, wobei ein Gemisch aus aromatischen Kohlenwasserstoffen eingesetzt wird. Hierbei können beispielsweise Gemische, welche Benzol und Toluol enthalten eingesetzt werden. Es ist aber auch möglich Gemische welche Benzol und Xylol, bzw. ein Xylol-Isomerengemisch, enthalten oder Gemische welche Benzol, Toluol und Xylol, bzw. ein Xylol-Isomerengemisch, enthalten einzusetzen. In Schritt a) wird der Gehalt an schwefelhaltigen aromatischen Verbindungen, wie z.B. Thiophen, auf ≤ 70 ppb, und der Gesamtschwefelgehalt auf insgesamt ≤ 200 ppb erniedrigt wird, und in Schritt b) der entschwefelte aromatische Kohlenwasserstoff oder das entschwefelte Gemisch aus aromatischen Kohlenwasserstoffen in Gegenwart eines geträgerten Rutheniumkatalysators und Wasserstoff zu dem entsprechenden cycloaliphtischen Kohlenwasserstoff oder dem entsprechenden Gemisch aus entsprechenden cycloaliphtischen Kohlenwasserstoffen reduziert. Im Fall von Benzol erhält man also als Hydrierungsprodukt Cyclohexan, aus Toluol Methylcyclohexan und aus Xylol das jeweils korrespondierende Dimethylcyclohexan, bzw. aus einem Xylol-Isomerengemisch das entsprechende Dimethylcyclohexan-Isomerengemisch, welches destillativ gereinigt werden kann.

Es existieren zahlreiche Verfahren zur Hydrierung von Benzol zu Cyclohexan. Diese Hydrierungen werden überwiegend an Nickel- und Platinkatalysatoren in der Flüssig- oder Gasphase durchgeführt (siehe hier unter anderem US 3,597,489, US 2,898,387, GB-A-1157356, GB-A-1097983, US2003/0106841 GB 799,396). Typischerweise wird dabei zunächst in einem ersten Reaktor der Großteil des Benzols zu Cyclohexan hydriert und anschließend in einem oder mehreren nachgeschalteten Rektoren die unumgesetzte Menge an Benzol zu Cyclohexan umgesetzt.

Die stark exotherme Hydrierreaktion erfordert eine sorgfältige Temperatur- und Verweilzeitkontrolle, um vollständigen Umsatz bei hoher Selektivität zu erreichen. Insbesonders muss eine signifikante Bildung von Methylcyclopentan unterdrückt werden; die bevorzugt bei höheren Temperaturen abläuft. Typische Cyclohexan-Spezifikationen fordern einen Benzol-Restgehalt <100 ppm und einen Methylcyclopentan-Gehalt <200 ppm. Ebenfalls ist auch der Gehalt an n-Parafinen (wie z.B. n-Pentan, n-Hexan) kritisch. Diese unerwünschten Verbindungen entstehen ebenfalls bevorzugt bei höheren Hydriertemperaturen und lassen sich ebenso wie Methylcyclopentan nur durch aufwendige Trennoperationen (wie z.B. Extraktion, Rektifikation oder Verwendung von Molekularsieben, wie in GB 1,341,057 beschrieben) von dem gewünschten Cyclohexan abtrennen. Auch der eingesetzte Katalysator hat einen starken Einfluss auf das Ausmaß der Bildung von unerwünschten Nebenkomponenten, wie Methylcyclohexan, n-Hexan, n-Pentan etc.

In Anbetracht dieses Hintergrundes ist es erstrebenswert, die Hydrierung bei möglichst niedrigen Temperaturen durchzuführen. Dies wird aber andererseits limitiert, da in Abhängigkeit von der Art des verwendeten Hydrierkatalysators erst ab höheren Temperaturen eine genügend hohe Hydrieraktivität des Katalysators erreicht wird, die seinerseits ausreichend ist eine wirtschaftliche Raum-Zeit-Ausbeute zu erhalten.

Die für die Benzolhydrierung eingesetzten Nickel- und Platinkatalysatoren weisen zudem eine Reihe von Nachteilen auf. Nickelkatalysatoren sind sehr empfindlich gegenüber schwefelhaltigen Verunreinigungen in Benzol, so dass entweder sehr reines Benzol zur Hydrierung eingesetzt werden muss, oder, wie in GB 1,104,275 beschrieben, im Hauptreaktor ein Platin-Katalysator eingesetzt wird, der einen höheren Schwefelgehalt toleriert, und so den Nachreaktor, der einen Nickelkatalysator enthält, schützt.

Eine andere Möglichkeit besteht darin, den Hydrierkatalysator mit Rhenium zu dotieren, wie in GB 1,155,539 beschrieben, oder Ionenaustauscher in den Hydrierkatalysator zu inkorporieren, wie dies in GB 1,144,499 offenbart wird. Die Herstellung derartiger Katalysatoren ist jedoch aufwendig und teuer.

Platinkatalysatoren weisen weniger Nachteile als Nickelkatalysatoren, sind jedoch sehr teuer.

Als Alternative wird daher in der jüngeren Literatur auf Ruthenium-haltige Katalysatoren zur Hydrierung von Benzol zu Cylohexan verwiesen.

In SU 319 582 werden Ruthenium-Suspensionskatalysatoren, die mit Palladium, Platin oder Rhodium dotiert sind, zur Herstellung von Cyclohexan aus Benzol beschrieben. Diese sind jedoch aufgrund des verwendeten Palladium, Platin oder Rhodium sehr teuer und zudem ist bei Suspensionskatalysatoren die Aufarbeitung und Wiedergewinnung des Katalysators sowohl aufwendig wie teuer.

In US 3,917,540 werden Al₂O₃-geträgerte Katalysatoren zur Herstellung von Cyclohexan aus Benzol beschrieben. Diese enthalten als Aktivmetall ein Edelmetall aus der VIII. Nebengruppe des Periodensystems sowie ein Alkalimetall und Technetium oder Rhenium. Weiterhin werden in US 3,244,644 η-Al₂O₃-geträgerte Ruthenium-Hydrierkatalysatoren beschrieben, die sich auch zur Hydrierung von Benzol eignen sollen. Diese Katalysatoren enthalten jedoch mindestens 5% Aktivmetall. Weiterhin ist die Herstellung von η-Al₂O₃ sowohl aufwendig wie teuer.

Weiterhin wird in WO 00/63142 u.a. die Hydrierung von unsubstituierten Aromaten unter Verwendung eines Katalysators, der als Aktivmetall mindestens ein Metall der VIII. Nebengruppe des Periodensystems enthält und welcher auf einem Träger mit Makroporen aufgetragen ist, beschrieben. Als Aktivmetall eignet sich insbesondere Ruthenium und als Träger insbesondere entsprechende Aluminiumoxide und Zirkoniumdioxide.

Ein Vorteil dieser Verfahren liegt in den vergleichsweise günstigen Kosten für Ruthenium, das als Aktivmetall für den Katalysator verwendet wird, im Vergleich zu den Kosten die durch andere Hydriermetalle, wie Palladium, Platin oder Rhodium, entstehen. Nachteilig ist aber auch hier, dass diese Ruthenium-Katalysatoren empfindlich gegenüber Schwefelverunreinigungen sind.

Aus EP 600 406 ist bekannt, dass ungesättigte Kohlenwasserstoffe, wie Alkene (wie z.B. Ethen), die mit Thiophen verunreinigt sind, entschwefelt werden können, indem man den ungesättigten Kohlenwasserstoff in Gegenwart eines Kupfer-Zink-Entschwefelungsmittel, das ein Kupfer/Zink-Atomverhältnis von 1 : etwa 0,3 - 10 besitzt, und das durch ein Co-Präzipationsverfahren erhältlich ist, mit 0, 01 bis 4 Vol.-% Wasserstoff behandelt. Insbesondere wird betont, dass die Menge an Wasserstoff diese Werte nicht überschreiten sollte, denn dies führt zu einer unerwünschten Hydrierung der zu reinigenden ungesättigten Kohlenwasserstoffe.

Der vorliegenden Erfindung lag die primäre Aufgabe zugrunde ein Verfahren zur Hydrierung von aromatischen Kohlenwasserstoffen oder Gemischen hiervon, die schwefelhaltige aromatische Verbindungen enthalten, zu den entsprechenden Cycloaliphaten oder Gemischen hiervon, insbesondere von Benzol unter Erhalt von Cyclohexan, bereitzustellen, und das es ermöglicht die Cycloaliphaten, oder die Gemische hiervon, mit sehr hoher Selektivität und Raum-Zeit-Ausbeute zu erhalten.

Demgemäss betrifft die vorliegende Erfindung ein Verfahren zur Umsetzung eines aromatischen Kohlenwasserstoffes, welcher schwefelhaltige aromatische Verbindungen enthält, oder eines Gemisches aus aromatischen Kohlenwasserstoffen, welches schwefelhaltige aromatische Verbindungen enthält, wobei in einem ersten Schritt schwefelhaltige aromatische Verbindungen ggf. in Gegenwart von Wasserstoff entfernt werden (Schritt a); diese Entschwefelung wird in Gegenwart eines Kupfer-Zink-Entschwefelungsmittels, das ein Kupfer : Zink-Atomverhältnis von 1 : 0,3 bis 1 : 10 besitzt und durch ein Copräzipitationverfahren erhältlich ist, durchgeführt. In einem zweiten Schritt wird der so erhaltene aromatische Kohlenwasserstoff oder das so erhaltene Gemisch an aromatischen Kohlenwasserstoffen in Gegenwart eines geträgerten Rutheniumkatalysators und Wasserstoff zu dem entsprechenden Cycloaliphaten, oder Gemischen hiervon, hydriert (Schritt b), wobei der Katalysator auf einem Träger aufgebracht ist, der Meso- und/oder Makroporen aufweist.

In einer bevorzugten Ausführungsform wird als aromatischer Kohlenwasserstoff Benzol eingesetzt, welcher zu Cyclohexan in Gegenwart von Wasserstoff hydriert wird.

In einer weiteren bevorzugten Ausführungsform wird ein Gemisch an aromatischen Kohlenwasserstoffen eingesetzt, welches zu dem entsprechenden Gemisch an Cycloaliphaten in Gegenwart von Wasserstoff hydriert wird. Als Gemische an aromatischen Kohlenwasserstoffen kommen hierbei solche in Betracht, die Benzol und Toluol, oder Benzol und Xylol, bzw. ein Xylol-Isomerengemisch, oder Benzol, Toluol und Xylol, bzw. ein Xylol-Isomerengemisch, enthalten. Bei der Hydrierung wird aus Benzol Cyclohexan, aus Toluol Methylcyclohexan und aus den Xylolen die entsprechenden Dimethylcyclohexane erhalten.

In Schritt a) wird der aromatische Kohlenwasserstoff, oder das Gemisch aromatischer Kohlenwasserstoffe, der als Verunreinigung schwefelhaltige aromatische Verbindungen enthält, entschwefelt. Als schwefelhaltige aromatische Verunreinigungen kommen besonders Thiophen, Benzothiophen, Dibenzothiophen oder entsprechende alkylierte Derivate, insbesondere Thiophen in Betracht. Neben diesen schwefelhaltigen aromatischen Verbindungen können auch weitere schwefelhaltige Verunreinigungen, wie z.B. Schwefelwasserstoff, Mercaptane, wie Methylmercaptan, Tetrahydrothiophen, Disulfide, wie Dimethyldisulfid, COS oder CS₂, - nachfolgend nicht-aromatische Schwefelverbindungen genannt - in dem aromatischen Kohlenwasserstoff, oder dem Gemisch aromatischer Kohlenwasserstoffe, enthalten sein. Weiterhin können auch andere Verunreinigungen, wie Wasser, C₅-C₇-Alkane, wie z.B. n-Heptan, C₅-C₇-Alkene, wie z.B. Penten oder Hexen, wobei die Doppelbindung an jeder Stelle des Kohlenstoffskeletts stehen kann, C₅-C₇-Cycloalkane, wie z.B. Methylcyclopentan, Ethylcyclopentan, Dimethylcyclopentan, Cyclohexan, Methylcyclohexan, oder C₅-C₇-Cycloalkene, wie z. B. Cyclohexen, enthalten sein.

Der in einer besonderen Ausführungsform eingesetzte aromatische Kohlenwasserstoff, hat in der Regel eine Reinheit von > 98 Gew.-%, insbesondere > 99 Gew.-%, vorzugsweise > 99,5 Gew.-%, insbesonders bevorzugt > 99,9 Gew.-%. Wird ein Gemisch an aromatischen Kohlenwasserstoffen eingesetzt, so liegt der Anteil an aromatischen Kohlenwasserstoffen bei dem eingesetzten Gemisch bei > 98 Gew.-%, insbesondere > 99 Gew.-%, vorzugsweise > 99,5 Gew.-%, insbesonders bevorzugt > 99,9 Gew.-%. In beiden Fällen kann der Gehalt an schwefelhaltigen aromatischen Verunreinigungen bis zu 2 Gew.-ppm, vorzugsweise bis zu 1 Gew.-ppm betragen. Der Gehalt an Gesamtschwefelverunreinigungen kann bis zu 5 Gew.-ppm, vorzugsweise bis zu 3 Gew.-ppm, insbesondere bis zu 2 Gew.-ppm, im speziellen bis zu 1 Gew.-ppm betragen. Sonstige Verunreinigungen können bis zu 2 Gew.-%, vorzugsweise bis zu 0,5 Gew.-%, insbesondere bis zu 0,1 Gew.-% betragen. Wasser kann in dem aromatischen Kohlenwasserstoff oder in den entsprechenden Gemischen von aromatischen Kohlenwasserstoffen bis zu 0,1 Gew.-%, vorzugsweise bis zu 0,07 Gew.-%, inbesondere bis zu 0,05 Gew.-% enthalten sein.

Die Entschwefelung wird an einem Kupfer-Zink-Entschwefelungsmittel, ggf. in Gegenwart von Wasserstoff durchgeführt. Dieses Kupfer-Zink-Entschwefelungsmittel enthält mindestens Kupfer und Zink, wobei das Kupfer : Zink-Atomverhältnis im Bereich von 1 : 0,3 bis 1 : 10, vorzugsweise bei 1 : 0,5 bis 1 : 3 und insbesonders bei 1 : 0,7 bis 1: 1,5 liegt. Es wird durch ein Copräzipitationverfahren erhalten und kann in oxidierter als auch in reduzierter Form eingesetzt werden.

In einer besonderen Ausführungsform enthält das Kupfer-Zink-Entschwefelungsmittel mindestens Kupfer, Zink und Aluminium, wobei das Kupfer : Zink : Aluminium-Atomverhältnis im Bereich von 1 : 0,3 : 0.05 bis 1 : 10 : 2, vorzugsweise bei 1 : 0,6 : 0,3 bis 1 : 3 : 1 und insbesondere bei 1 : 0,7 : 0,5 bis 1 : 1,5 : 0,9 liegt.

Die Entschwefelungsmittel können nach verschiedenen Verfahren hergestellt werden. Beispielsweise kann eine wässrige Lösung, die eine Kupferverbindung, insbesondere eine wasserlösliche, wie z.B. Kupfernitrat oder Kupferacetat, und eine Zinkverbindung, insbesondere eine wasserlösliche, wie z.B. Zinknitrat oder Zinkacetat, enthält, mit einer wässrigen Lösung einer alkalischen Substanz (wie z.B. Natriumcarbonat, Kaliumcarbonat) unter Bildung eines Niederschlages miteinander vermischt werden (Copräzipitationsverfahren). Der gebildete Niederschlag wird abfiltriert, mit Wasser gewaschen oder erst gewaschen, dann filtriert und anschließend getrocknet. Dann calciniert man bei etwa 270 bis 400°C. Anschließend wird der erhaltene Feststoff in Wasser aufgeschlämmt, abfiltriert und getrocknet. Das so erhaltene Kupfer-Zink-Entschwefelungsmittel ("oxiderte Form") kann in dieser Form in die Entschwefelung eingesetzt werden.

In einer weiteren Ausführungsform ist es möglich das so erhaltene Mischoxid einer Wasserstoffreduktion zu unterwerfen. Diese wird bei etwa 150 bis 350°C, vorzugsweise bei etwa 150 bis 250°C, in Gegenwart von Wasserstoff durchgeführt, wobei der Wasserstoff durch ein Inertgas, wie z.B. Stickstoff, Argon, Methan, insbesondere Stickstoff, verdünnt wird, so dass der Wasserstoffgehalt 10 Vol.-% oder weniger, vorzugsweise 6 Vol.-% oder weniger, insbesondere 0,5 bis 4 Vol.-%, beträgt. Das so erhaltene Kupfer-Zink-Entschwefelungsmittel ("reduzierte Form") kann in dieser Form in die Entschwefelung eingesetzt werden.

Weiterhin kann das Kupfer-Zink-Entschwefelungsmittel auch Metalle enthalten, die zur Gruppe VIII des Periodensystems (wie Fe, Co, Ni, Ru, Rh, Pd, Os, Ir, Pt), zur Gruppe IB (wie Ag, Au) oder zur Gruppe VIB (wie Cr, Mo, W) gehören. Diese können hergestellt werden, indem die entsprechenden Metallsalze in die oben genannten Herstellverfahren zugegeben werden.

Weiterhin ist es möglich den nach der Calcinierung oder auch den nach der Wasserstoffbehandlung, erhaltenen Feststoff zu Tabletten oder in andere Formen zu formen oder zu extrudieren, wobei es hilfreich sein kann, Zusatzstoffe, wie z.B. Bindemittel, beispielsweise Graphit, hinzufügen.

In einer weiteren Ausgestaltungsform kann. eine Lösung, die eine Kupferverbindung, insbesondere eine wasserlösliche, wie z.B. Kupfernitrat oder Kupferacetat, eine Zinkverbindung, insbesondere eine wasserlösliche, wie z.B. Zinknitrat oder Zinkacetat, und eine Aluminiumverbindung, wie z.B. Aluminiumhydroxid, Aluminiumnitrat, Natriumaluminat enthält, mit einer wässrigen Lösung einer alkalischen Substanz, wie z.B. Natriumcarbonat, Kaliumcarbonat, unter Bildung eines Niederschlages miteinander vermischt werden (Copräzipitationsverfahren). Der gebildete Niederschlag wird abfiltriert, mit Wasser gewaschen oder erst gewaschen, dann filtriert und getrocknet. Dann calciniert man bei etwa 270 bis 400°C. Anschließend wird der erhaltene Feststoff in Wasser aufgeschlämmt, abfiltriert und getrocknet. Das so erhaltene Kupfer-Zink-Entschwefelungsmittel ("oxidierte Form") kann in dieser Form in die Entschwefelung eingesetzt werden.

In einer weiteren Ausführungsform ist es möglich das so erhaltene Mischoxid einer Wasserstoffreduktion zu unterwerfen. Diese wird bei etwa 150 bis 350°C, vorzugsweise bei etwa 150 bis 250°C, in Gegenwart von Wasserstoff durchgeführt, wobei der Wasserstoff durch ein Inertgas, wie z.B. Stickstoff, Argon, Methan, insbesondere Stickstoff, verdünnt wird, so dass der Wasserstoffgehalt 10 Vol.-% oder weniger, vorzugsweise 6 Vol.-% oder weniger, insbesondere 0,5 bis 4 Vol.-%, beträgt. Das so erhaltene Kupfer-Zink-Entschwefelungsmittel ("reduzierte Form") kann in dieser Form in die Entschwefelung eingesetzt werden.

Weiterhin kann das Kupfer-Zink-Entschwefelungsmittel auch Metalle enthalten, die zur Gruppe VIII des Periodensystems (wie Fe, Co, Ni, Ru, Rh, Pd, Os, Ir, Pt), zur Gruppe IB (wie Ag, Au) oder zur Gruppe VIB (wie Cr, Mo, W) gehören. Diese können hergestellt werden indem die entsprechenden Metallsalze in die oben genannten Herstellverfahren zugegeben werden.

Weiterhin ist es möglich den nach der Calcinierung oder auch den nach der Wasserstoffbehandlung, erhaltenen Feststoff zu Tabletten oder in andere Formen zu formen oder zu extrudieren, wobei es hilfreich sein kann, Zusatzstoffe, wie z.B. Bindemittel, beispielsweise Graphit, hinzufügen.

In einer weiteren Ausgestaltungsform kann die Copräzipitation pH-kontrolliert durchgeführt werden, indem man beispielsweise die Zulaufgeschwindigkeit der Salzlösungen so einstellt, dass während der Fällung ein pH-Wert von etwa 7 bis 7,5 eingehalten wird. Es ist auch möglich den Niederschlag, der bei der Fällung entsteht, nach dem Waschen einer Sprühtrocknung zu unterwerfen.

In einer weiteren Ausführungsform kann die Copräzipitation derart durchgeführt werden, dass die Kupferoxid-Zinkoxid-Komponenten aus wässrigen Lösungen der entsprechenden Salze (wie z.B. Nitrate oder Acetate) mit einer alkalisch reagierenden Substanz (wie z.B. Alkalicarbonat, Ammoniumcarbonat) in Gegenwart von kolloidal (als Gel oder Sol) verteilten Aluminiumoxid, Aluminiumhydroxid ausgefällt werden.

Die Calcinierung, die ggf. gewünschte Wasserstoffbehandlung und die Formgebung können wie oben beschrieben erfolgen.

Weiterhin ist es möglich kommerziell verfügbare Katalysatoren, wie z.B. den Katalysator R 3-12 von BASF oder G-132A von Süd-Chemie, einzusetzen.

In einer bevorzugten Ausführungsform wird das Kupfer-Zink-Entschwefelungsmittel in der reduzierten Form eingesetzt. Es kann von Vorteil sein, das Mischoxid, das nach den oben beschriebenen Verfahren erhalten wird einer Wasserstoffreduktion zu unterwerfen, die wie folgt ausgeführt werden kann (im nachfolgenden steht [Kat] für Katalystator):
1. Das Mischoxid wird auf 100 bis 140°C, insbesondere auf 120 ± 5°C bei einer Stickstoffstrom von 200 bis 400 Nm³/m³ _{[KAT]}•h, insbesondere von 300 ± 20 Nm³/m³ _{[KAT]}•h, erwärmt.
2. Zu Beginn der Reduktion werden zu dem oben genannten Stickstoffstrom 0,5 ± 0,1 Vol.-% Wasserstoff dosiert bis sich eine Temperaturerhöhung von 15 bis 20°C ergibt und diese konstant bleibt. Anschließend wird der Wasserstoffstrom auf 1,0 ± 0,1 Vol.-% Wasserstoff erhöht bis sich insgesamt eine Temperaturerhöhung von max. 30 ± 5°C ergibt und die Temperatur konstant bleibt.
3. Anschließend wird der Wasserstoffstrom auf 2,0 ± 0,2 Vol.-% erhöht, wobei die Temperatur des Katalysators nicht über 230°C, vorzugsweise 225°C steigen sollte.
4. Der Wasserstoffstrom wird nun auf 4,0 ± 0,4 Vol.-% erhöht und gleichzeitig die Temperatur des Stickstoffes auf 200 ± 10° erhöht, wobei auch hier die Temperatur des Katalysators nicht über 230°C, vorzugsweise 225°C steigen sollte.
5. Jetzt wird der Wasserstoffstrom auf 6,0 ± 0,6 Vol.-% erhöht und gleichzeitig die Temperatur des Katalysators bei 220 ± 10°C gehalten.
6. Anschließend wird bei einem Stickstoffstrom von 200 bis 400 Nm³/m³ _{[KAT]}•h, insbesondere von 300 ± 20 Nm³/m³ _{[KAT]}•h, auf unter 50°C abgekühlt, wobei die Kühlrate 50 ± 5 K/h nicht überschreiten sollte.

Das so erhaltene Kupfer-Zink-Entschwefelungsmittel liegt nun in der "reduzierten Form" vor und kann so eingesetzt werden. Er kann aber auch unter Inertgas bis zu seinem Einsatz aufbewahrt werden. Weiterhin ist es auch möglich das Kupfer-Zink-Entschwefelungsmittel in einem inerten Lösungsmittel aufzubewahren. Von Fall zu Fall kann es von Vorteil sein, das Kupfer-Zink-Entschwefelungsmittel in seiner oxidierten Form aufzubewahren und die Aktivierung "just-in-time" durchzuführen. In diesen Zusammenhang kann es auch von Vorteil sein, vor der Aktivierung einen Trocknungsschritt durchzuführen. Hierbei wird das calcinierte, in oxidischer Form vorliegende Kupfer-Zink-Entschwefelungsmittel in einem Stickstoffstrom von 200 bis 400 Nm³/m³ _{[KAT]}•h, insbesondere von 300 ± 20 Nm³/m³_{[KAT]}•h, auf 180 bis 220°C, insbesondere auf 200 ± 10°C erwärmt, wobei die Heizrate nicht 50 K/h überschreiten sollte. Sobald das Wasser entfernt ist kann auf 100 bis 140°C, insbesondere auf 120 ± 5°C, abgekühlt werden, wobei die Kühlrate nicht 50 K/h überschreiten sollte und die Aktivierung, wie voranstehend beschrieben, durchgeführt werden.

In einer insbesonders bevorzugten Ausführungsform wird ein Kupfer-Zink-Entschwefelungsmittel verwendet, welches 35 bis 45 Gew.-%, vorzugsweise 38 bis 41 Gew.-%, Kupferoxid, 35 bis 45 Gew.-%, vorzugsweise 38 bis 41 Gew.-%, Zinkoxid, und 10 bis 30 Gew.-%, vorzugsweise 18 bis 24 Gew.-%, Aluminiumoxid sowie ggf. weitere Metalloxide enthält.

In einer außerordentlich bevorzugten Ausführungsform wird ein Kupfer-Zink-Entschwefelungsmittel verwendet, welches 38 bis 41 Gew.-%, Kupferoxid, 38 bis 41 Gew.-% Zinkoxid, und 18 bis 24 Gew.-%, Aluminiumoxid enthält.

Diese Kupfer-Zink-Entschwefelungsmittel sind aus entsprechenden calcinierten Mischoxiden nach den oben genannten Herstellverfahren erhältlich.
In einer Ausführungsform wird die Entschwefelung des aromatischen Kohlenwasserstoffes, oder des Gemisches von aromatischen Kohlenwasserstoffen, vorzugsweise von Benzol, an dem Kupfer-Zink-Entschwefelungsmittel in oxidierter Form ohne Zusatz von Wasserstoff durchgeführt.

In einer weiteren Ausführungsform wird die Entschwefelung des aromatischen Kohlenwasserstoffes, oder des Gemisches von aromatischen Kohlenwasserstoffen, vorzugsweise von Benzol, an dem Kupfer-Zink-Entschwefelungsmittel in oxidierter Form in Gegenwart von Wasserstoff durchgeführt.

In einer weiteren Ausführungsform wird die Entschwefelung des aromatischen Kohlenwasserstoffes, oder des Gemisches von aromatischen Kohlenwasserstoffen, vorzugsweise von Benzol, an dem Kupfer-Zink-Entschwefelungsmittel in reduzierter Form ohne Zusatz von Wasserstoff durchgeführt.

In einer weiteren Ausführungsform wird die Entschwefelung des aromatischen Kohlenwasserstoffes, oder des Gemisches von aromatischen Kohlenwasserstoffen, vorzugsweise von Benzol, an dem Kupfer-Zink-Entschwefelungsmittel in reduzierter Form in Gegenwart von Wasserstoff durchgeführt.

Üblicherweise wird die Entschwefelung in einem Temperaturbereich von 40 bis 200°C, besonders bei 50 bis 180°C, insbesondere bei 60 bis 160°C, vorzugsweise bei 70 bis 120°C, bei einen Druck von 1 bis 40 bar, besonders bei 1 bis 32 bar, vorzugsweise bei 1,5 bis 5 bar, insbesondere bei 2,0 bis 4,5 bar durchgeführt. Die Entschwefelung kann in Gegenwart von Inertgasen, wie z.B. Stickstoff, Argon oder Methan, durchgeführt werden. In der Regel wird jedoch die Entschwefelung ohne Zugabe von Inertgasen durchgeführt.

Üblicherweise setzt man - soweit gewünscht- hierbei Wasserstoff mit einer Reinheit von ≥ 99,8 Vol.-%, insbesondere von ≥ 99,9 Vol.-%, vorzugsweise von ≥ 99,95 Vol.-% ein. Diese Reinheitsgrade gelten analog für den Wasserstoff, der bei den ggf. durchgeführten Aktivierungen der Katalysatoren verwendet wird.

Üblicherweise liegt das Gewichtsverhältnis von aromatischem Kohlenwasserstoff, oder des Gemisches von aromatischen Kohlenwasserstoffen, zu Wasserstoff im Bereich von 40 000 : 1 bis1 000 : 1, besonders im Bereich von 38 000 : 1 bis 5 000 : 1, insbesonders im Bereich von 37 000 : 1 bis 15 000 : 1, vorzugsweise im Bereich 36 000 : 1 bis 25 000 : 1, im speziellen im Bereich von 35 000 : 1 bis 30 000 :1.

In der Regel liegt die LHSV ("Liquid Hourly Space Velocity") im Bereich von 0,5 bis 10 kg aromatischer Kohlenwasserstoff pro Volumenteil Katalysator und Stunde (kg/(m³_{[Kat]}•h)), insbesondere im Bereich von 1 bis 8 kg/(m³_{[Kat]}•h) vorzugsweise im Bereich von 2 bis 6 kg/(m³_{[Kat]}•h).

Der so entschwefelte aromatische Kohlenwasserstoff, oder das Gemisch von aromatischen Kohlenwasserstoffen, vorzugsweise Benzol, hat nunmehr einen Gehalt von aromatischen Schwefelverbindungen von höchstens 70 ppb, vorzugsweise von höchstens 50 ppb, und der Gesamtschwefelgehalt liegt bei insgesamt ≤ 200 ppb, vorzugsweise ≤ 150 ppb, insbesondere ≤ 100 ppb.

Die voranstehend beschriebenen Entschwefelungsmittel ermöglichen es auch, Chlor, Arsen und/oder Phosphor bzw. entsprechende Chlor-, Arsen und/oder Phosphorhaltige Verbindungen aus dem aromatischen Kohlenwasserstoff, oder dem Gemisch von aromatischen Kohlenwasserstoffen, zu reduzieren oder zu entfernen.

Die Entschwefelung des aromatischen Kohlenwasserstoffes, oder des Gemisches von aromatischen Kohlenwasserstoffen, kann in einem oder in mehreren parallel oder in Reihe geschalteten Reaktoren erfolgen. Diese Reaktoren werden üblicherweise in Sumpffahrweise betrieben, wobei das Gas und die Flüssigkeit im Gleichstrom oder im Gegenstrom, vorzugsweise im Gleichstrom geführt werden. Es besteht aber auch die Möglichkeit die Reaktoren in Rieselfahrweise zu betreiben, wobei das Gas und die Flüssigkeit im Gleichstrom oder im Gegenstrom, vorzugsweise im Gegenstrom, geführt werden.

Falls es notwendig ist, kann das Entschwefelungsmittel auch aus dem Reaktor wieder entfernt werden. Liegt das Entschwefelungsmittel in reduzierter Form vor, so kann es von Vorteil sein, das Entschwefelungsmittel vor dem Ausbau einer Oxidation zu unterwerfen. Als Oxidationsmittel werden Sauerstoff, oder Gemische von Sauerstoff mit einem oder mehreren inerten Gasen, wie z.B. Luft, verwendet. Die Oxidation erfolgt nach dem Fachmann bekannten üblichen Verfahren. Beispielsweise kann die Oxidation wie folgt durchgeführt werden:
1. Das Entschwefelungsmittel wird zuerst mit einem Stickstoffstrom von 200 bis 400 Nm³/m³ _{[KAT]}•h, insbesondere von 300 ± 20 Nm³/m³ _{[KAT]}•h gespült.
2. Zu Beginn der Oxidation werden zu dem oben genannten Stickstoffstrom 5 bis 10 Nm³/m³ _{[KAT]}•h, insbesondere 7 ± 1 Nm³/m³ _{[KAT]}•h, Luft zudosiert, wobei sich die Temperatur um etwa 50 °C erhöht. Anschließend wird der Luftstrom innerhalb eines Zeitraums von 0,5 h bis 2,0 h, vorzugsweise 1 ± 0,2 h, auf 10 bis 18 Nm³/m³ _{[KAT]}•h, insbesondere auf 14 ± 1 Nm³/m³ _{[KAT]}•h, erhöht und für 6 bis 10 h, vorzugsweise 8 ± 0,5 h, gehalten.
3. Anschließend wird der Luftstrom innerhalb eines Zeitraums von 0,5 h bis 2,0 h, vorzugsweise 1 ± 0,2 h, auf 20 bis 35 Nm³/m³ _{[KAT]}•h, insbesondere auf 28 ± 2 Nm³/m³ _{[KAT]}•h, erhöht, wobei die Temperatur des Entschwefelungsmittels nicht über 230°C, vorzugsweise 225°C steigen sollte und für 3 bis 5 h, vorzugsweise 4 ± 0,5 h, gehalten.
4. Dann wird der Luftstrom auf 120 bis 180 Nm³/m³[_{KAT]}•h, insbesondere auf 150 ± 10 Nm³/m³ _{[KAT]}•h, erhöht und zugleich der Stickstoffstrom auf ebenfalls 120 bis 180 Nm³/m³ _{[KAT]}•h, insbesondere auf 150 ± 10 Nm³/m³ _{[KAT]}•h, erniedrigt, wobei die Temperatur des Entschwefelungsmittels nicht über 230°C, vorzugsweise 225°C steigen sollte. Diese Fahrweise wird solange beibehalten, bis die Temperatur sinkt und der Gehalt an Sauerstoff im Abgas dem Eingangsgehalt entspricht.
5. Anschließend wird bei einem Stickstoffstrom auf Null zurückgefahren und der Luftstrom auf 200 bis 400 Nm³/m³ _{[KAT]}•h, insbesondere auf 300 ± 20 Nm³/m³ _{[KAT]}•h erhöht. Diese Fahrweise wird in der Regel etwa 1 Stunde beibehalten, bis die Oxidation vollständig ist.

Das so erhaltene Kupfer-Zink-Entschwefelungsmittel kann nun ausgebaut werden.

Im Schritt b) wird nun der entschwefelte aromatische Kohlenwasserstoff, oder das Gemisch von aromatischen Kohlenwasserstoffen, in Gegenwart eines geträgerten Rutheniumkatalysators zu dem entsprechenden Cycloaliphaten, bzw. den entsprechenden Gemischen von Cycloaliphaten, hydriert, wobei der Katalysator auf einem Träger aufgebracht ist, der Meso- und/oder Makroporen aufweist.

Als Träger können prinzipiell alle Träger eingesetzt werden, die Makroporen aufweisen, d.h. Träger, die ausschließlich Makroporen aufweisen als auch solche, die neben Makroporen auch Mesoporen und/oder Mikroporen enthalten. Die Begriffe "Makroporen", "Mesoporen" und "Mikroporen" werden im Rahmen der vorliegenden Erfindung so verwendet, wie sie in Pure Appl. Chem. 46, 71 (1976) definiert sind, nämlich als Poren, deren Durchmesser oberhalb von 50 nm (Makroporen) oder deren Durchmesser zwischen 2 uns 50 nm liegt (Mesoporen) oder deren Durchmesser < 2nm (Mikroporen) liegt.

Insbesondere eignen sich als Träger entsprechende Aktivkohlen, Siliciumcarbide, Aluminiumoxide, Siliciumoxide, Titandioxide, Zirkoniumdioxide, oder auch Gemische hiervon. Bevorzugt werden entsprechende Aluminiumoxide, Zirkoniumdioxide oder Siliciumoxide, insbesondere γ-Aluminiumoxid oder Siliciumoxide, eingesetzt.
- In einer besonderen Ausgestaltungsform wird ein auf γ-Aluminiumoxid-geträgerter Ruthenium-Katalysator eingesetzt.

Im allgemeinen beträgt der Gehalt an Ruthenium 0,01 bis 30 Gew.-%, vorzugsweise 0,01 bis 5 Gew.-% und insbesondere 0,1 bis 1,5 Gew.-% jeweils bezogen auf das Gesamtgewicht des Katalysators.

In einer bevorzugten Ausführungsform wird ein geträgerter Ruthenium-Katalysator verwendet, wobei der Träger einen mittleren Porendurchmesser von mindestens 50 nm und eine BET-Oberfäche von höchstens 30 m²/g aufweist und wobei die Mengen an Ruthenium 0,01 bis 30 Gew.-%, bezogen auf das Gesamtgewicht des Katalysators, beträgt. Insbesondere bevorzugt sind geträgerte Ruthenium-Katalysatoren, wobei der Träger einen mittleren Porendurchmesser von 100 nm bis 200 µm und eine BET-Oberfläche von maximal 15 m²/g aufweist.

In einer weiteren bevorzugten Ausführungsform wird ein geträgerter Ruthenium-Katalysator verwendet, wobei die Mengen an Ruthenium 0,01 bis 30 Gew.-%, bezogen auf das Gesamtgewicht des Katalysators, beträgt und wobei 10 bis 50 % des Porenvolumens des Trägers von Makroporen mit einem Porendurchmesser in Bereich von 50 nm bis 10 000 nm gebildet werden und 50 bis 90 % des Porenvolumens des Trägers von Mesoporen mit einem Porendurchmesser im Bereich von 2 bis 50 nm gebildet werden und wobei sich die Summe der Anteile der Porenvolumina zu 100 % addiert. (Die Bestimmung des mittleren Porendurchmessers und der Porengrößenverteilung erfolgt durch Hg-Porosimetrie, insbesondere nach DIN 66133.)

Die geträgerten Rutheniumkatalysatoren werden durch Auftragen des Ruthenium auf den Träger hergestellt. Dies kann in der Regel durch Tränken des Trägers mit wässrigen Rutheniumsalzlösungen erfolgen oder durch Besprühen des Trägers mit entsprechender Rutheniumsalzlösung. Als Rutheniumsalze eignen sich die Nitrate, Nitrosylnitrate, Halogenide, Carbonate, Carboxylate, Acetylacetonate, Chlorkomplexe, Nitritokomplexe oder Aminkomplexe, insbesondere das Nitrat und das Nitrosylnitrat.

Die mit der Rutheniumsalzlösung getränkten bzw. beschichteten Träger werden anschließend in der Regel bei Temperaturen von 100 bis 150°C getrocknet und wahlweise bei Temperaturen von 200 bis 600°C, vorzugsweise bei 350 bis 450°C calciniert.

Der so erhaltene, calcinierte, geträgerte Rutheniumkatalysator wird nun durch Behandlung in einem Gasstrom, der freien Wasserstoff enthält, bei Temperaturen von 30 bis 600°C, vorzugsweise bei 150 bis 450°C, aktiviert. In der Regel besteht der Gasstrom aus 50 bis 100 Vol.-% Wasserstoff und bis zu 50 Vol.-% Stickstoff.

Üblicherweise wird die Rutheniumsalzlösung in einer solchen Menge auf den Träger aufgebracht, dass der Gehalt an Ruthenium, 0,01 bis 30 Gew.-%, vorzugsweise 0,01 bis 5 Gew.-%, besondere 0,01 bis 1 Gew.-% und insbesonders 0,05 bis 1 Gew.-% jeweils bezogen auf das Gesamtgewicht des Katalysators, beträgt.

In einer besonderen Ausführungsform werden Trägermaterialien verwendet, die makroporös sind und einen mittleren Porendurchmesser von mindestens 50 nm, vorzugsweise von mindestens 100 nm, insbesonders von mindestens 500 nm aufweisen und deren Oberfläche nach BET bei höchstens 30 m²/g, vorzugsweise bei höchstens 15 m²/g, besondere bei höchstens 5 m²/g und insbesonders bei 0,5 bis 3 m²/g liegt. Der mittlere Porendurchmesser dieser Träger liegt vorzugsweise bei 100 nm bis 200 µm, vorzugsweise bei 500 nm bis 50 µm (Die Oberfläche des Trägers wird bestimmt nach dem BET-Verfahren durch N₂-Adsorption, insbesondere nach DIN 66131).

Die Metalloberfläche auf dem so erhaltenen geträgerten Rutheniumkatalysators beträgt 0,01 bis 10 m²/g, bevorzugt 0,05 bis 5 m²/g und insbesondere 0,05 bis 3 m²/g. (Die Metalloberfläche wird mittels des von J. Lemaire et al. in "Charaterization of Heterogeneous Catalysts", Hrsg. Francis Delanney, Marcel Dekker, New York 1984, S. 310- 324 beschriebenen Chemiesorptionsverfahrens bestimmt.)

Das Verhältnis der Metalloberfläche zu der Katalysatorträgeroberfläche beträgt hierbei höchstens 0,05, insbesondere höchstens 0,005.

Vorzugsweise kann die Porenverteilung des Trägers annähernd bimodal sein. Vorzugsweise hat eine derartige bimodale Porendurchmesserverteilung Maxima bei etwa 600 nm und bei etwa 20 µm.

In einer weiteren bevorzugten Ausführungsform werden Trägermaterialien verwendet, welche Makroporen und Mesoporen aufweisen. Insbesondere weisen diese eine Porenverteilung der Gestalt aus, dass 5 bis 50 %, vorzugsweise 10 bis 45 %, besonders 10 bis 30 % und insbesonders 15 bis 25 % des Porenvolumens von Makroporen, mit einem Porendurchmesser im Bereich von 50 nm bis 10 000 nm, und 50 bis 95 %, vorzugsweise 55 bis 90 %, besonders 70 bis 90 % und insbesonders 75 bis 85 % des Porenvolumens von Mesoporen, mit einem Porendurchmesser von 2 bis 50 nm, gebildet werden. Die Summe der Anteile der Porenvolumina addiert sich hierbei zu 100 %.

Das Gesamtporenvolufnen der hierbei verwendeten Träger beträgt 0,05 bis 1,5 cm³/g, vorzugsweise 0,1 bis 1,2 cm³/g und insbesonders 0,3 bis 1,0 cm³/g.

Der mittlere Porendurchmesser der hierbei verwendeten Träger liegt bei 5 bis 20 nm, vorzugsweise bei 8 bis 15 nm und insbesonders bei 9 bis 12 nm. (Die Bestimmung des mittleren Porendurchmessers erfolgt durch Hg-Porosimetrie, insbesondere nach DIN 66133.)

Die Oberfläche der hierbei verwendeten Träger liegt im Bereich von 50 bis 500 m²/g, vorzugsweise im Bereich von 200 bis 350 m²/g und insbesonders im Bereich von 200 bis 300 m²/g. (Die Oberfläche des Trägers wird bestimmt nach dem BET-Verfahren durch N₂-Adsorption, insbesondere nach DIN 66131).
- In einer weiteren Ausgestaltungsform kann ein Schalenkatalysator enthaltend als Aktivmetall Ruthenium alleine oder zusammen mit mindestens einem weiteren Metall der Nebengruppen IB, VIIB oder VIII des Periodensystems der Elemente (CAS-Version), aufgebracht auf einen Träger enthaltend Siliciumdioxid als Trägermaterial verwendet werden

Dieser Schalenkatalysator ist dann dadurch gekennzeichnet, dass die Menge des Aktivmetalls < 1 Gew.-%, bevorzugt 0,1 bis 0,5 Gew.-%, besonders bevorzugt 0,25 bis 0,35 Gew.-%, bezogen auf das Gesamtgewicht des Katalysators, beträgt und mindestens 60 Gew.-%, besonders bevorzugt 80 Gew.-% des Aktivmetalls, bezogen auf die Gesamtmenge des Aktivmetalls, in der Schale des Katalysators bis zu einer Eindringtiefe von 200 µm vorliegen. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Weitere Informationen bezüglich den vorstehend genannten Messverfahren und Techniken sind zum Beispiel "Spectroscopy in Catalysis" von J.W. Niemantsverdriet, VCH, 1995 offenbart.

Der Schalenkatalysator zeichnet sich dadurch aus, dass die überwiegende Menge des Aktivmetalls in der Schale bis zu einer Eindringtiefe von 200 µm, also nahe der Oberfläche des Schalenkatalysators vorliegt. Dagegen liegt im Inneren (Kern) des Katalysators keine oder nur eine sehr geringe Menge des Aktivmetalls vor.

Bevorzugt ist ein Schalenkatalysator, in dem kein Aktivmetall im Inneren des Katalysators nachgewiesen werden kann, d.h. Aktivmetall liegt nur in der äußersten Schale, zum Beispiel in einer Zone bis zu einer Eindringtiefe von 100 -200 µm, vor.

Der Schalenkatalysator zeichnet sich in einer weiteren besonders bevorzugten Ausführungsform dadurch aus, dass im (FEG)-TEM (Field Emission Gun -Transmission Electron Microscopy) mit EDXS nur in den äußersten 200 µm, bevorzugt 100 µm, ganz besonders bevorzugt 50 µm (Eindringtiefe) Aktivmetallteilchen nachgewiesen werden können. Teilchen kleiner 1 nm können nicht nachgewiesen werden.

Als Aktivmetall kann Ruthenium alleine oder zusammen mit mindestens einem weiteren Metall der Nebengruppen IB, VIIB oder VIII des Periodensystems der Elemente (CAS-Version) eingesetzt werden. Neben Ruthenium geeignete weitere Aktivmetalle sind z.B. Platin, Rhodium, Palladium, Iridium, Cobalt oder Nickel oder ein Gemisch aus zwei oder mehr davon. Unter den ebenfalls verwendbaren Metallen der Nebengruppen IB und/oder VIIB des Periodensystems der Elemente sind z.B. Kupfer und/oder Rhenium geeignet. Bevorzugt wird Ruthenium alleine als Aktivmetall oder zusammen mit Platin oder Iridium in dem Schalenkatalysator eingesetzt; ganz besonders bevorzugt wird Ruthenium alleine als Aktivmetall eingesetzt.

Der Schalenkatalysator zeigt die vorstehend erwähnte sehr hohe Aktivität bei einer geringen Beladung mit Aktivmetall, die < 1 Gew.-%, bezogen auf das Gesamtgewicht des Katalysators, beträgt. Bevorzugt beträgt die Menge des Aktivmetalls in dem Schalenkatalysator 0,1 bis 0,5 Gew.-%, besonders bevorzugt 0,25 bis 0,35 Gew.-%. Es wurde gefunden, dass die Eindringtiefe des Aktivmetalls in das Trägermaterial von der Beladung des Katalysators mit Aktivmetall abhängig ist. Bereits bei einer Beladung des Katalysators mit 1 Gew.-% oder mehr, z.B. bei einer Beladung mit 1,5 Gew.-%, ist im Inneren des Katalysators, d.h. in einer Eindringtiefe von 300 bis 1000 µm eine wesentliche Menge Aktivmetall vorhanden, die die Aktivität des Hydrierkatalysators, insbesondere die Aktivität über einen langen Hydrierzeitraum, beeinträchtigt, insbesondere bei schnellen Reaktionen, wobei Wasserstoffmangel im Inneren des Katalysators (Kern) auftreten kann.

In einer Ausführungsform des Schalenkatalysators liegen mindestens 60 Gew.-% des Aktivmetalls, bezogen auf die Gesamtmenge des Aktivmetalls, in der Schale des Katalysators bis zu einer Eindringtiefe von 200 µm vor. Bevorzugt liegen in dem Schalenkatalysator mindestens 80 Gew.-% des Aktivmetalls, bezogen auf die Gesamtmenge des Aktivmetalls, in der Schale des Katalysators bis zu einer Eindringtiefe von 200 µm vor. Ganz besonders bevorzugt ist ein Schalenkatalysator, in dem kein Aktivmetall im Inneren des Katalysators nachgewiesen werden kann, d.h. Aktivmetall liegt nur in der äußersten Schale, zum Beispiel in einer Zone bis zu einer Eindringtiefe von 100 - 200 µm , vor. In einer weiteren bevorzugten Ausführungsform liegen 60 Gew.-%, bevorzugt 80 Gew.-%, bezogen auf die Gesamtmenge des Aktivmetalls, in der Schale des Katalysators bis zu einer Eindringtiefe von 150 µm vor. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Zur Ermittlung der Eindringtiefe der Aktivmetallteilchen werden mehrere Katalysatorteilchen (z.B. 3, 4 oder 5) quer zur Strangachse (wenn der Katalysator in Form von Strängen vorliegt) angeschliffen. Mittels Linescans werden dann die Profile der Aktivmetall/Si Konzentrationsverhältnisse erfasst. Auf jeder Messlinie werden mehrere zum Beispiel 15-20 Messpunkte in gleichen Abständen gemessen; die Messfleckgröße beträgt circa 10 µm•10 µm. Nach Integration der Aktivmetallmenge über die Tiefe kann die Häufigkeit des Aktivmetalls in einer Zone bestimmt werden.

Ganz besonders bevorzugt beträgt die Menge des Aktivmetalls, bezogen auf das Konzentrationsverhältnis von Aktivmetall zu Si, an der Oberfläche des Schalenkatalysators, 2 bis 25 %, bevorzugt 4 bis 10 %, besonders bevorzugt 4 bis 6 %, ermittelt mittels SEM EPMA - EDXS. Die Oberflächeanalyse erfolgt mittels Bereichsanalysen von Bereichen von 800 µm x 2000 µm und mit einer Informationstiefe von circa 2 µm. Die Elementzusammensetzung wird in Gew.-% (normiert auf 100 %) bestimmt. Das mittlere Konzentrationsverhältnis (Aktivmetall/Si) wird über 10 Messbereiche gemittelt.

Unter der Oberfläche des Schalenkatalysators ist im Sinne der vorliegenden Anmeldung die äußere Schale des Katalysators bis zu einer Eindringtiefe von circa 2 µm zu verstehen. Diese Eindringtiefe entspricht der Informationstiefe bei der voranstehend erwähnten Oberflächenanalyse.

Ganz besonders bevorzugt ist ein Schalenkatalysator, worin die Menge des Aktivmetalls, bezogen auf das Gewichtsverhältnis von Aktivmetall zu Si (Gew./Gew. in %), an der Oberfläche des Schalenkatalysators 4 bis 6 % beträgt, in einer Eindringtiefe von 50 µm 1,5 bis 3 % und im Bereich von 50 bis 150 µm Eindringtiefe 0,5 bis 2 %, ermittelt mittels SEM EPMA (EDXS), beträgt. Die genannten Werte stellen gemittelte Werte dar.

Des Weiteren nimmt die Größe der Aktivmetallteilchen bevorzugt mit zunehmender Eindringtiefe ab, ermittelt mittels (FEG)-TEM-Analyse.

Das Aktivmetall liegt in dem Schalenkatalysator bevorzugt teilweise oder vollständig kristallin vor. In bevorzugten Fällen kann in der Schale des Schalenkatalysators mittels SAD (Selected Area Diffraction) oder XRD (X-Ray Diffraction) feinstkristallines Aktivmetall nachgewiesen werden.

Der Schalenkatalysator kann zusätzlich Erdalkalimetallionen (M²⁺), also M = Be, Mg, Ca, Sr und/oder Ba, insbesondere Mg und/oder Ca, ganz besonders Mg enthalten. Der Gehalt an Erdalkalimetallion(en) (M²⁺) im Katalysator beträgt bevorzugt 0,01 bis 1 Gew.-%, insbesondere 0,05 bis 0,5 Gew.-%, ganz besonders 0,1 bis 0,25 Gew.-%, jeweils bezogen auf das Gewicht des Siliciumdioxid-Trägermaterials.

Ein wesentlicher Bestandteil der Katalysatoren ist das Trägermaterial auf Basis von Siliciumdioxid, im Allgemeinen amorphem Siliciumdioxid. Unter dem Begriff "amorph" versteht man in diesem Zusammenhang, dass der Anteil kristalliner Siliciumdioxid-Phasen weniger als 10 Gew.-% des Trägermaterials ausmacht. Die zur Herstellung der Katalysatoren verwendeten Trägermaterialien können allerdings Überstrukturen aufweisen, die durch regelmäßige Anordnung von Poren im Trägermaterial gebildet werden.

Als Trägermaterialien kommen grundsätzlich amorphe Siliciumdioxid-Typen in Betracht, die wenigstens zu 90 Gew.-% aus Siliciumdioxid bestehen, wobei die verbleibenden 10 Gew.-%, vorzugsweise nicht mehr als 5 Gew.-%, des Trägermaterials auch ein anderes oxidisches Material sein können, z.B. MgO, CaO, TiO₂, ZrO₂, Fe₂O₃ und/oder Alkalimetalloxid.

In einer bevorzugten Ausführungsform der Erfindung ist das Trägermaterial halogenfrei, insbesondere chlorfrei, d. h. der Gehalt an Halogen im Trägermaterial beträgt weniger als 500 Gew.-ppm, z.B. im Bereich von 0 bis 400 Gew.-ppm. Somit ist ein Schalenkatalysator bevorzugt, der weniger als 0,05 Gew.-% Halogenid (ionenchromatographisch bestimmt), bezogen auf das Gesamtgewicht des Katalysators, enthält. Bevorzugt sind Trägermaterialien, die eine spezifische Oberfläche im Bereich von 30 bis 700 m²/g, vorzugsweise 30 bis 450 m²/g, (BET-Oberfläche nach DIN 66131) aufweisen.

Geeignete amorphe Trägermaterialien auf Basis von Siliciumdioxid sind dem Fachmann geläufig und kommerziell erhältlich (siehe z.B. O.W. Flörke, "Silica" in Ullmann's Encyclopedia of Industrial Chemistry 6th Edition on CD-ROM). Sie können sowohl natürlichen Ursprungs als auch künstlich hergestellt worden sein. Beispiele für geeignete amorphe Trägermaterialien auf Basis von Siliciumdioxid sind Kieselgele, Kieselgur, pyrogene Kieselsäuren und Fällungskieselsäuren. In einer bevorzugten Ausführungsform der Erfindung weisen die Katalysatoren Kieselgele als Trägermaterialien auf.

Je nach Ausgestaltung der Erfindung kann das Trägermaterial unterschiedliche Gestalt aufweisen. Bei Einsatz des Schalenkatalysators in Katalysatorfestbetten verwendet man üblicherweise Formkörper aus dem Trägermaterial, die z.B. durch Extrudieren, Strangpressen oder Tablettieren erhältlich sind und die z.B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 0,5 mm bis 25 mm. Häufig werden Katalysatorstränge mit Strangdurchmessern von 1,0 bis 5 mm und Stranglängen von 2 bis 25 mm eingesetzt. Mit kleineren Strängen können im Allgemeinen höhere Aktivitäten erzielt werden; jedoch zeigen diese oft keine ausreichende mechanische Stabilität im Hydrierverfahren. Daher werden ganz besonders bevorzugt Stränge mit Strangdurchmessern im Bereich von 1,5 bis 3 mm eingesetzt.

Die Herstellung der Schalenkatalysatoren erfolgt bevorzugt dadurch, dass man zunächst das Trägermaterial mit einer Lösung von Ruthenium(III)-Acetat alleine oder zusammen mit einer Lösung von mindestens einem weiteren Salz von Metallen der Nebengruppen IB, VIIB oder VIII des Periodensystems der Elemente (CAS-Version) ein- oder mehrfach tränkt, den erhaltenen Feststoff trocknet und anschließend reduziert, wobei die Lösung des mindestens einen weiteren Salzes von Metallen der Nebengruppen IB, VIIB oder VIII des Periodensystems der Elemente in einem oder mehreren gemeinsamen Tränkschritten gemeinsam mit der Lösung von Ruthenium(III)-Acetat oder in einem oder mehreren Tränkschritten getrennt von der Lösung von Ruthenium(III)-Acetat aufgebracht werden kann. Die einzelnen Verfahrensschritte sind nachstehend genauer beschrieben.

Die Herstellung des Schalenkatalysators umfassend die Schritte:
1) ein- oder mehrfache Tränkung des Trägermaterials enthaltend Siliciumdioxid mit einer Lösung von Ruthenium(III)-Acetat alleine oder zusammen mit einer Lösung von mindestens einem weiteren Salz von Metallen der Nebengruppen IB, VIIB oder VIII des Periodensystems der Elemente (CAS-Version);
2) anschließendes Trocknen;
3) anschließende Reduktion;
wobei die Lösung des mindestens einen weiteren Salzes von Metallen der Nebengruppen IB, VIIB oder VIII des Periodensystems der Elemente in einem oder mehreren Tränkschritten gemeinsam mit der Lösung von Ruthenium(III)-Acetat oder in einem oder mehreren Tränkschritten getrennt von der Lösung von Ruthenium(III)-Acetat aufgebracht werden kann.

In oben genannten Schritt 1) erfolgt eine ein- oder mehrfache Tränkung des Trägermaterials enthaltend Siliciumdioxid mit einer Lösung von Ruthenium(III)-Acetat alleine oder zusammen mit mindestens einem weiteren gelösten Salz von Metallen der Nebengruppen IB, VIIB oder VIII des Periodensystems der Elemente (CAS-Version). Da die Menge an Aktivmetall in dem Schalenkatalysator sehr gering ist, erfolgt in einer bevorzugten Ausführungsform eine einfache Tränkung. Ruthenium(III)-Acetat bzw. die Salze von Metallen der Nebengruppen IB, VIIB oder VIII des Periodensystems der Elemente stellen dabei Aktivmetallprekursor dar. Insbesondere bei Verwendung von Ruthenium(III)-Acetat als Prekursor können Schalenkatalysatoren erhalten werden, die sich unter anderem dadurch auszeichnen, dass sich der wesentliche Teil des Aktivmetalls, bevorzugt Ruthenium alleine, in dem Schalenkatalysator bis zu einer Eindringtiefe von 200 µm befindet. Das Innere des Schalenkatalysators weist kein oder nur wenig Aktivmetall auf.

Geeignete Lösungsmittel zur Bereitstellung der Lösung von Ruthenium(III)-Acetat bzw. der Lösung von mindestens einem weiteren Salz von Metallen der Nebengruppen IB, VIIB oder VIII des Periodensystems der Elemente sind Wasser oder auch Mischungen von Wasser bzw. Lösemittel mit bis zu 50 Vol.-% eines oder mehrerer mit Wasser bzw. Lösemittel mischbarer organischer Lösungsmittel, z.B. Mischungen mit C₁-C₄-Alkanolen wie Methanol, Ethanol, n-Propanol oder Isopropanol. Wässrige Essigsäure oder Eisessig können ebenfalls verwendet werden. Alle Mischungen sollten so gewählt werden, dass eine Lösung oder Phase vorliegt. Bevorzugte Lösungsmittel sind Essigsäure, Wasser oder Mischungen hiervon. Besonders bevorzugt wird eine Mischung von Wasser und Essigsäure als Lösungsmittel eingesetzt, da Ruthenium(III)-Acetat üblicherweise gelöst in Essigsäure oder Eisessig vorliegt. Ruthenium(III)-Acetat kann jedoch auch als Feststoff nach Lösen verwendet werden. Der Katalysator auch kann ohne Verwendung von Wasser hergestellt werden.

Die Lösung des mindestens einen weiteren Salzes von Metallen der Nebengruppen IB, VIIB oder VIII des Periodensystems der Elemente kann in einem oder mehreren Tränkschritten gemeinsam mit der Lösung von Ruthenium(III)-Acetat oder in einem oder mehreren Tränkschritten getrennt von der Lösung von Ruthenium(III)-Acetat aufgebracht werden. Das bedeutet, dass das Tränken mit einer Lösung erfolgen kann, die Ruthenium(III)-Acetat sowie mindestens ein weiteres Salz von Metallen der Nebengruppen IB, VIIB oder VIII des Periodensystems der Elemente aufweist. Das Tränken mit dieser Lösung kann ein- oder mehrfach erfolgen. Es ist jedoch ebenfalls möglich, dass zunächst mit einer Ruthenium(III)-Acetat- Lösung getränkt wird und anschließend in einem getrennten Tränkschritt mit einer Lösung, die mindestens ein weiteres Salz von Metallen der Nebengruppen IB, VIIB oder VIII des Periodensystems der Elemente aufweist, getränkt wird. Die Reihenfolge der Tränkschritte kann auch umgekehrt sein. Es ist ebenfalls möglich, dass einer der beiden Tränkschritte oder beide Tränkschritte einmal oder mehrfach in beliebiger Reihenfolge wiederholt werden. Nach jedem Tränkschritt wird üblicherweise getrocknet.

Geeignete Salze von weiteren Metallen der Nebengruppen IB, VIIB oder VIII des Periodensystems der Elemente, die in dem Tränkschritt eingesetzt werden können, sind z.B. Nitrate, Acetonate und Acetate, wobei Acetate bevorzugt sind.

Besonders bevorzugt erfolgt eine Tränkung mit einer Lösung von Ruthenium(III)-Acetat allein in einem Tränkschritt.

Das Tränken des Trägermaterials kann in unterschiedlicher Weise erfolgen und richtet sich in bekannter Weise nach der Gestalt des Trägermaterials. Beispielsweise kann man das Trägermaterial mit der Prekursor-Lösung besprühen oder spülen oder das Trägermaterial in der Prekursor-Lösung suspendieren. Beispielsweise kann man das Trägermaterial in einer wässrigen Lösung des Aktivmetallprekursors suspendieren und nach einer gewissen Zeit vom wässrigen Überstand abfiltrieren. Über die aufgenommene Flüssigkeitsmenge und die Aktivmetall-Konzentration der Lösung kann dann der Aktivmetallgehalt des Katalysators in einfacher Weise gesteuert werden. Das Tränken des Trägermaterials kann beispielsweise auch dadurch erfolgen, dass man den Träger mit einer definierten Menge der Lösung des Aktivmetallprekursors behandelt, die der maximalen Flüssigkeitsmenge entspricht, die das Trägermaterial aufnehmen kann. Zu diesem Zweck kann man beispielsweise das Trägermaterial mit der erforderlichen Flüssigkeitsmenge besprühen. Geeignete Apparaturen hierfür sind die zum Vermengen von Flüssigkeiten mit Feststoffen üblicherweise verwendeten Apparate (siehe Vauck/Müller, Grundoperationen chemischer Verfahrenstechnik, 10. Auflage, Deutscher Verlag für Grundstoffindustrie, 1994, S. 405 ff.), beispielsweise Taumeltrockner, Tränktrommeln, Trommelmischer, Schaufelmischer und dergleichen. Monolithische Träger werden üblicherweise mit den wässrigen Lösungen des Aktivmetallprekursors gespült.

Die zum Tränken eingesetzten Lösungen sind vorzugsweise halogenarm, insbesondere chlorarm, d.h. sie enthalten kein oder weniger als 500 Gew.-ppm, insbesondere weniger als 100 Gew.-ppm Halogen, z.B. 0 bis < 80 Gew.-ppm Halogen, bezogen auf das Gesamtgewicht der Lösung.

Die Konzentration des Aktivmetallprekursors in den Lösungen richtet sich naturgemäß nach der aufzubringenden Menge an Aktivmetallprekursor und der Aufnahmekapazität des Trägermaterials für die Lösung und beträgt < 20 Gew.-%, bevorzugt 0,01 bis 6 Gew.-%, besonders bevorzugt 0,1 bis 1,1 Gew.%, bezogen auf die Gesamtmasse der eingesetzten Lösung.

Im Schritt 2) wird die Trocknung durchgeführt. Diese kann nach den üblichen Verfahren der Feststofftrocknung unter Einhaltung der unten genannten Temperaturobergrenzen erfolgen. Die Einhaltung der Obergrenze der Trocknungstemperaturen ist für die Qualität, d.h. die Aktivität des Katalysators wichtig. Ein Überschreiten der unten angegebenen Trocknungstemperaturen führt zu einem deutlichen Verlust an Aktivität. Ein Calcinieren des Trägers bei höheren Temperaturen, z.B. oberhalb 300°C oder gar 400°C, wie es im Stand der Technik vorgeschlagen wird, ist nicht nur überflüssig sondern wirkt sich auch nachteilig auf die Aktivität des Katalysators aus. Zur Erreichung hinreichender Trocknungsgeschwindigkeiten erfolgt die Trocknung bevorzugt bei erhöhter Temperatur, bevorzugt bei ≤ 180°C, besonders bei ≤ 160°C, und bei wenigstens 40°C, insbesondere wenigstens 70°C, speziell wenigstens 100°C, ganz besonders im Bereich von 110°C bis 150°C.

Die Trocknung des mit dem Aktivmetallprekursor getränkten Feststoffs erfolgt üblicherweise unter Normaldruck wobei zur Förderung der Trocknung auch ein verminderter Druck angewendet werden kann. Häufig wird man zur Förderung der Trocknung einen Gasstrom über bzw. durch das zu trocknende Gut leiten, z.B. Luft oder Stickstoff.

Die Trocknungsdauer hängt naturgemäß von dem gewünschten Grad der Trocknung und der Trocknungstemperatur ab und liegt bevorzugt im Bereich von 1 h bis 30 h, vorzugsweise im Bereich von 2 bis 10 h.

Vorzugsweise führt man die Trocknung des behandelten Trägermaterials soweit, dass der Gehalt an Wasser bzw. an flüchtigen Lösungsmittelbestandteilen vor der anschließenden Reduktion weniger als 5 Gew.-%, insbesondere nicht mehr als 2 Gew.-%, bezogen auf das Gesamtgewicht des Feststoffs ausmacht. Die angegebenen Gewichtsanteile beziehen sich hierbei auf den Gewichtsverlust des Feststoffs, bestimmt bei einer Temperatur von 160°C, einem Druck von 1 bar und einer Dauer von 10 Min. Auf diese Weise kann die Aktivität der verwendeten Katalysatoren weiter gesteigert werden.

In Schritt 3) wird der nach dem Trocknen erhaltenen Feststoff in seine katalytisch aktive Form übergeführt. Dies erfolgt durch Reduzieren des Feststoffs bei Temperaturen im Bereich von im Allgemeinen 150°C bis 450°C, bevorzugt 250°C bis 350°C in an sich bekannter Weise.

Zu diesem Zweck bringt man den nach den Trocknen erhaltenen Feststoff bei den oben angegebenen Temperaturen mit Wasserstoff oder einer Mischung aus Wasserstoff und einem Inertgas in Kontakt. Der Wasserstoffabsolutdruck ist für das Ergebnis der Reduktion von untergeordneter Bedeutung und kann z.B. im Bereich von 0,2 bar bis 1,5 bar variiert werden. Häufig erfolgt die Hydrierung des Katalysatormaterials bei Wasserstoffnormaldruck im Wasserstoffstrom. Vorzugsweise erfolgt die Reduktion unter Bewegen des Feststoffs, beispielsweise durch Reduzieren des Feststoffs in einem Drehrohrofen oder einem Drehkugelofen. Auf diese Weise kann die Aktivität der Katalysatoren weiter gesteigert werden. Der eingesetzte Wasserstoff ist vorzugsweise frei von Katalysatorgiften, wie CO- und S- enthaltenden Verbindungen, z.B. H₂S, COS und anderen.

Die Reduktion kann auch mittels organischer Reduktionsreagenzien wie Hydrazin, Formaldehyd, Formiaten oder Acetaten erfolgen.

Im Anschluss an die Reduktion kann der Katalysator zur Verbesserung der Handhabbarkeit in bekannter Weise passiviert werden, z.B. indem man den Katalysator kurzfristig mit einem Sauerstoff-haltigen Gas, z.B. Luft, vorzugsweise jedoch mit einer 1 bis 10 Vol.-% Sauerstoff enthaltenden Inertgasmischung, behandelt. Auch CO₂ oder CO₂/O₂-Mischungen können hier angewendet werden.

Der aktive Katalysator kann auch unter einem inerten organischen Lösungsmittel, z.B. Ethylenglykol, aufbewahrt werden.

Zur Herstellung des Schalenkatalysators kann in einer weiteren Durchführungsform der Aktivmetall-Katalysator-Vorläufer, z.B. wie oben hergestellt oder wie in WO-A2-02/100538 (BASF AG) beschrieben hergestellt, mit einer Lösung eines oder mehrerer Erdalkalimetall(II)salze imprägniert werden.

Bevorzugte Erdalkalimetall(II)salze sind entsprechende Nitrate, wie insbesondere Magnesiumnitrat und Calciumnitrat.

Bevorzugtes Lösungsmittel für die Erdalkalimetall(II)salze in diesem Imprägnierungsschritt ist Wasser. Die Konzentration des Erdalkalimetall(II)salzes im Lösungsmittel beträgt z.B. 0,01 bis 1 mol/Liter.

Zum Beispiel wird der in einem Rohr eingebaute Aktivmetall/SiO₂-Katalysato mit einem Strom einer wässrigen Lösung des Erdalkalimetallsalzes kontaktiert. Der zu imprägnierende Katalysator kann auch mit einer überstehenden Lösung des Erdalkalimetallsalzes behandelt werden.

Bevorzugt findet so eine Sättigung des Aktivmetall/SiO₂-Katalysators insbesondere seiner Oberfläche, mit dem oder den Erdalkalimetallion/en statt.

Überschüssiges Erdalkalimetallsalz und nicht immobilisierte Erdalkalimetallionen wird/werden vom Katalysator gespült (H₂O-Spülung, Katalysatorwaschung).

Für vereinfachte Handhabung, z.B. Einbau in einem Reaktorrohr, kann der Katalysator nach der Imprägnierung getrocknet werden. Die Trocknung kann dafür z.B. in einem Ofen bei < 200°C, z.B. bei 50 bis 190°C, besonders bevorzugt bei < 140°C, z. B. bei 60 bis 130°C, durchgeführt werden.

Dieses Imprägnierungsverfahren kann ex situ oder in situ durchgeführt werden: Ex situ heißt vor Einbau des Katalysators in den Reaktor, in situ bedeutet im Reaktor (nach dem Katalysatoreinbau).

In einer Verfahrensvariante kann eine Imprägnierung des Katalysators mit Erdalkalimetallionen auch in situ dadurch erfolgen, dass der Lösung des zu hydrierenden aromatischen Substrats (Edukts) Erdalkalimetallionen, z.B. in Form von gelösten Erdalkalimetallsalzen, zugegeben werden. Dazu wird z.B. die entsprechende Menge Salz zunächst in Wasser gelöst und dann dem in einem organischen Lösungsmittel gelöstem Substrat zugegeben.

Gemäß einer Variante kann im Hydrierverfahren der Katalysator in Kombination mit dem zu hydrierenden Substrats, das eine Erdalkalimetallionen-haltige Lösung enthält,eingesetzt wird.Dabei liegt der Gehalt an Erdalkalimetallionen in dem zu hydrierenden Substrats im Allgemeinen 1 bis 100 Gew.-ppm, insbesondere 2 bis 10 Gew.-ppm.

Herstellungsbedingt liegt das Aktivmetall in den Katalysatoren als metallisches Aktivmetall vor.

Durch die Verwendung halogenfreier, insbesondere chlorfreier, Aktivmetallprekursor und Lösungsmittel bei der Herstellung des Schalenkatalysators liegt der Halogenidgehalt, insbesondere Chloridgehalt, der Schalenkatalysatoren zudem unterhalb 0,05 Gew.-% (0 bis < 500 Gew.-ppm, z.B. im Bereich von 0 - 400 Gew.-ppm), bezogen auf das Gesamtgewicht des Katalysators.

Der Chloridgehalt wird z.B. mit der unten beschriebenen Methode ionenchromatographisch bestimmt.

In einer ausgewählten Variante ist bevorzugt, dass das mittels ²⁹Si-Festkörper-NMR bestimmte prozentuale Verhältnis der Q₂ - und Q₃ - Strukturen Q₂/Q₃ kleiner als 25, bevorzugt kleiner als 20, besonders bevorzugt kleiner als 15 ist, z.B. im Bereich von 0 bis 14 oder 0,1 bis 13 liegt. Dies bedeutet auch, dass der Kondensationsgrad des Silikas in dem verwendeten Träger besonders hoch ist.

Die Identifikation der Qₙ - Strukturen (n = 2, 3, 4) und die Bestimmung des prozentualen Verhältnisses erfolgt mittels ²⁹Si-Festkörper-NMR.

Qₙ = Si(OSi)ₙ(OH)₄₋ₙ mit n = 1, 2, 3 oder 4.

Man findet Qₙ für n = 4 bei -110,8 ppm, n = 3 bei -100,5 ppm und n = 2 bei -90,7 ppm (Standard: Tetramethylsilan) (Q₀ und Q₁ wurden nicht identifiziert). Die Analyse wird unter den Bedingungen des "magic angle spinning" bei Raumtemperatur (20°C) (MAS 5500 Hz) mit Kreispolarisation (CP 5 ms) und unter Verwendung von dipolarer Entkopplung der ¹H durchgeführt. Wegen der teilweisen Überlagerung der Signale werden die Intensitäten über eine Linienformanalyse ausgewertet. Die Linienformanalyse wurde mit einem Standard Softwarepaket der Fa. Galactic Industries durchgeführt, wobei eine "least square fit" iterativ berechnet wurde.

Vorzugsweise enthält das Trägermaterial nicht mehr als 1 Gew.-% und insbesondere nicht mehr als 0,5 Gew.-% und insbesondere < 500 Gew.-ppm an Aluminiumoxid, gerechnet als Al₂O₃.

Da die Kondensation des Silikas auch durch Aluminium und Eisen beeinflusst werden kann, ist die Konzentration an Al(III) und Fe(II und/oder III) in Summe bevorzugt kleiner als 300 Gew.-ppm, besonders bevorzugt kleiner 200 Gew.-ppm, und liegt z.B. im Bereich von 0 bis 180 Gew.-ppm.

Der Anteil an Alkalimetalloxid resultiert bevorzugt aus der Herstellung des Trägermaterials und kann bis zu 2 Gew.-% betragen. Häufig beträgt er weniger als 1 Gew.-%. Geeignet sind auch Alkalimetalloxid-freie Träger (0 bis < 0,1 Gew.-%). Der Anteil an MgO, CaO, TiO₂ bzw. an ZrO₂ kann bis zu 10 Gew.-% des Trägermaterials ausmachen und beträgt vorzugsweise nicht mehr als 5 Gew.-%. Geeignet sind aber auch Trägermaterialien, die keine nachweisbaren Mengen dieser Metalloxide enthalten (0 bis < 0,1 Gew.-%).

Weil Al(III) und Fe(II und/oder III) in Silica eingebaut acide Zentren ergeben können, ist es bevorzugt, dass eine Ladungskompensierung bevorzugt mit Erdalkalimetallkationen (M²⁺, M = Be, Mg, Ca, Sr, Ba) im Träger vorliegt. Dies bedeutet, dass das Gewichtsverhältnis von M(II) zu (Al(III) + Fe(II und/oder III)) größer ist als 0,5, bevorzugt > 1, besonders bevorzugt größer als 3. (Die römischen Zahlen in Klammern hinter dem Elementsymbol bedeuten die Oxidationsstufe des Elements.)

Die Hydrierung des entschwefelten aromatischen Kohlenwasserstoffes, oder des Gemisches von entschwefelten aromatischen Kohlenwasserstoffen, vorzugsweise Benzol, an den voranstehend beschriebenen geträgerten Rutheniumkatalysatoren, zu dem Cycloaliphaten, oder dem entsprechenden Gemisch an Cycloaliphaten, vorzugsweise Cyclohexan, in Gegenwart von Wasserstoff kann in der Flüssigphase oder in der Gasphase durchgeführt werden. Bevorzugt wird das Hydrierverfahren in der Flüssigphase durchgeführt - in der Regel bei einer Temperatur von 50 bis 250°C, vorzugsweise bei 60 bis 200°C, insbesondere bei 70 bis 170°C. Die dabei verwendeten Drücke liegen im Bereich von 1 bis 200 bar, vorzugsweise bei 10 bis 50 bar, insbesondere bei 19 bis 40 bar und speziell bei 25 bis 35 bar.

Üblicherweise setzt man bei der Hydrierung Wasserstoff mit einer Reinheit von ≥ 99,8 Vol.-%, insbesondere von ≥ 99,9 Vol.-%, vorzugsweise von ≥ 99,95 Vol.-% ein.

Besonders bevorzugt erfolgt dabei eine vollständige Hydrierung des aromatischen Kohlenwasserstoffes, oder des Gemisches von aromatischen Kohlenwasserstoffen, wobei unter vollständiger Hydrierung ein Umsatz der zu hydrierenden Verbindung von im Allgemeinen ≥ 98 %, bevorzugt > 99 %, besonders bevorzugt > 99,5 %, ganz besonders bevorzugt > 99,9 %, insbesondere >99,99 % und speziell >99,995 % zu verstehen ist.

Üblicherweise liegt das Gewichtsverhältnis von aromatischem Kohlenwasserstoff, oder des Gemisches von aromatischen Kohlenwasserstoffen, zu Wasserstoff im Bereich von 8 : 1 bis 5:1, vorzugsweise von 7,7 : 1 bis 5,5 : 1, insbesondere bei 7,6 : 1 bis 6 : 1 und speziell bei 7,5 : 1 bis 6,5 : 1..

Die Hydrierung des entschwefelten aromatischen Kohlenwasserstoffes oder Gemischen von entschwefelten aromatischen Kohlenwasserstoffen, kann in einem oder in mehreren in Reihe oder parallel geschalteten Reaktoren, die vorzugsweise in Rieselfahrweise betrieben werden, durchgeführt werden. Hierbei werden das Gas und die Flüssigkeit im Gleichstrom oder im Gegenstrom, vorzugsweise im Gleichstrom geführt. Es ist aber auch möglich die in Reihe geschalteten Reaktoren in Sumpffahrweise zu betreiben.

In der Regel liegt die LHSV ("Liquid Hourly Space Velocity") im Bereich von 0,1 bis 10 kg aromatischer Kohlenwasserstoff pro Volumenteil Katalysator und Stunde (kg/(m³_{[Kat]}•h)), vorzugsweise im Bereich von 0,3 bis 1,5 kg/(m³_{[Kat]}•h). Die Berieselungsdichte liegt üblicherweise im Bereich von 20 bis 100 m³ aromatischer Kohlenwasserstoff pro angeströmte Querschnittsfläche des Katalysatorbettes und Stunde (m³/m²•h), vorzugsweise im Bereich von 60 bis 80 m³/m²•h.

Es kann von Vorteil sein in einem ersten Reaktor einen Umsetzungsgrad vom aromatischen Kohlenwasserstoff von 95 bis 99,5 % zu erreichen und in einem nachgeschalteten Reaktor einen Umsetzungsgrad von > 99,9 %, insbesondere > 99,99 %, vorzugsweise > 99,995 %. In einem derartigen Fall ist in der Regel das Verhältnis der Volumina der Katalysatorschüttungen von Hauptreaktor zu nachgeschalteten Reaktor im Bereich von 20 : 1 bis 3 : 1, insbesondere im Bereich 15 : 1 bis 5 : 1.

In einer weiteren Ausgestaltungsform kann der Hauptreaktor in Walzfahrweise betrieben werden. Das Walzverhältnis (Verhältnis von Zulauf in kg/h zu Rückführungsstrom in kg/h) liegt üblicherweise im Bereich von 1 : 5 bis 1 : 100, vorzugsweise im Bereich von 1 : 10 bis 1 : 50, bevorzugt im Bereich 1 : 15 bis 1 : 35. Hierbei ist es auch möglich die bei der Reaktion gebildete Wärme partiell oder vollständig abzuführen, indem der Rückführungsstrom über einen Wärmetauscher geleitet wird.

In einer weiteren Ausgestaltungsform kann der Nachreaktor auch in den Hauptreaktor integriert sein.

Von Fall zu Fall kann es auch notwendig werden, den Hydrierkatalysator, aufgrund nachlassender Aktivität, zu regenerieren. Dies erfolgt nach den für Edelmetallkatalysatoren wie Rutheniumkatalysatoren üblichen, dem Fachmann bekannten Methoden. Hier sind z.B. die Behandlung des Katalysators mit Sauerstoff wie in der BE 882 279 beschrieben, die Behandlung mit verdünnten, halogenfreien Mineralsäuren, wie in der US 4,072,628 beschrieben, oder die Behandlung mit Wasserstoffperoxid, z. B. in Form wässriger Lösungen mit einem Gehalt von 0,1 bis 35 Gew.-%, oder die Behandlung mit anderen oxidierenden Substanzen, vorzugsweise in Form halogenfreier Lösungen zu nennen. Üblicherweise wird man den Katalysator nach der Reaktivierung und vor dem erneuten Einsatz mit einem Lösungsmittel, z. B. Wasser, spülen.

Das gemäß des Verfahren erhaltene Reaktionsprodukt, also der Cycloaliphat, oder das Gemisch aus entsprechenden Cycloaliphaten, kann in einem Schritt c) weiter aufgereinigt werden.

Für den Fall, dass als Edukt ein aromatischer Kohlenwasserstoff eingesetzt und der entsprechende Cycloaliphat erhalten wird, dann kann das erhaltene Reaktionsprodukt einer Reindestillation unterworfen werden um ggf. entstandene Nebenprodukte, wie Leichtsieder bezüglich des entsprechenden Cycloaliphaten, z.B. n-Hexan und n-Pentan, oder auch Schwersieder abzutrennen. Wird beispielsweise Benzol als Edukt eingesetzt, so kann das erhaltene Cyclohexan als Verunreinigungen beispielsweise n-Hexan und n-Pentan enthalten, die als Leichtsieder abgetrennt werden können. Als Schwersieder kann ggf. Methylcyclohexan in Betracht kommen, das ebenfalls destillativ abgetrennt werden kann. Bei der Reindestillation kann das Rein-Cyclohexan über einen Seitenabzug an der Kolonne gewonnen werden, während die Leichtsiederkomponenten am Kopf und Schwersiederkomponenten am Sumpf abgezogen werden. Alternativ kann die Aufreinigung des Produktes auch in einer Kolonne mit Trennwand erfolgen, wobei hier das Rein-Cyclohexan auf der Höhe der Trennwand abgezogen wird.

Wird ein Gemisch an aromatischen Kohlenwasserstoffen als Edukt eingesetzt so werden die einzelnen Komponenten des entstehenden Cycloaliphatengemisches destillativ getrennt und ggf. weitere Verunreinigungen destillativ abgetrennt.

Die bei der exothermen Hydrierung entstehende Reaktionswärme kann ggf. bei entsprechender Wahl des Druckniveaus der Destillation dazu genutzt werden, den Verdampfer der Destillationskolonne zu betreiben. Dazu kann der heiße Reaktionsaustrag direkt in den Kolonnenverdampfer eingeleitet werden oder ggf. ein Sekundärmedium aufgeheizt (z. B. Erzeugung von Dampf) und in den Kolonnenverdampfer eingeleitet werden.

Die Teilschritte des Verfahrens als auch das Gesamtverfahren können kontinuierlich, semikontinuierlich oder diskontinuierlich durchgeführt werden.

Mit Hilfe des erfindungsgemäßen Verfahrens können somit hydrierte Produkte erhalten werden, die keine oder sehr geringe Restgehalte der zu hydrierenden Ausgangsprodukte enthalten.

Weiterhin betrifft die vorliegende Erfindung ein Verfahren zur Entschwefelung eines aromatischen Kohlenwasserstoffes, welcher schwefelhaltige aromatische Verbindungen enthält, ggf. in Gegenwart von Wasserstoff, wie voranstehend in Schritt a) beschrieben wird.

### REGENERIERUNGSSCHRITT

Bei Hydrierverfahren, in denen die oben dargestellten Katalysatoren eingesetzt werden, ist nach einer gewissen Katalysatorstandzeit eine Desaktivierung zu beobachten. Ein solcher deaktivierter Rutheniumkatalysator kann durch Spülen in den Zustand der ursprünglichen Aktivität zurückgeführt werden. Die Aktivität lässt sich bis auf > 90 %, vorzugsweise > 95 %, mehr bevorzugt > 98 %, insbesondere > 99 %, meist bevorzugt > 99,5 % des Ursprungswertes wieder herstellen. Die Desaktivierung wird auf Spuren beziehungsweise Reste an dem Katalysator adsorbierten Wasser zurückgeführt. Dies lässt sich überraschenderweise durch das Spülen mit Inertgas rückgängig machen. Das erfindungsgemäße Regenerierungsverfahren lässt sich somit auch als Trocknung des Katalysators oder Entfernen von Wasser von diesem bezeichnen.

"Spülen" bedeutet, dass der Katalysator mit Inertgas in Kontakt gebracht wird. Normalerweise wird dazu durch geeignete, dem Fachmann bekannte konstruktive Maßnahmen das Inertgas über den Katalysator geleitet.

Das Spülen mit Inertgas wird bei einer Temperatur von ungefähr 10 bis 350°C, bevorzugt von ungefähr 50 bis 250°C, besonders bevorzugt von ungefähr 70 bis 180°C, am meisten bevorzugt von ungefähr 80 bis 130°C durchgeführt.

Die beim Spülen angelegten Drücke betragen 0,5 bis 5 bar, vorzugsweise 0,8 bis 2 bar, insbesondere 0,9 bis 1,5 bar.

Erfindungsgemäß wird die Behandlung des Katalysators vorzugsweise mit einem Inertgas durchgeführt. Bevorzugte Inertgase umfassen Stickstoff, Kohlendioxid, Helium, Argon, Neon und Mischungen daraus. Am meisten bevorzugt ist Stickstoff.

Gemäß einer besonderen Ausführungsform der Erfindung wird das erfindungsgemäße Verfahren des Regenerierens ohne Ausbau des Katalysators in demselben Reaktor durchgeführt, in dem die Hydrierung stattgefunden hat. In besonders vorteilhafter Weise wird das Spülen des Katalysators gemäß der vorliegenden Erfindung bei Temperaturen und Drücken im Reaktor durchgeführt, die entsprechend oder ähnlich der Hydrierungsreaktion sind, wodurch eine nur sehr kurze Unterbrechung des Reaktionsprozesses resultiert.

Gemäß der vorliegenden Erfindung wird das Spülen mit Inertgas mit einem Volumenstrom von 20 bis 200 Nl/h, bevorzugt mit einem Volumenstrom von 50 bis 200 Nl/h pro Liter Katalysator durchgeführt.

Das Spülen mit Inertgas wird bevorzugt über eine Zeitdauer von 10 bis 50 Stunden, besonders bevorzugt von 10 bis 20 Stunden, durchgeführt. Beispielsweise beträgt die errechnete Trockenzeit des Katalysatorbettes einer großtechnischen Cyclohexan-Produktionsanlage mit einer angenommenen Feuchte von 2 beziehungsweise 5 Gew.-% näherungsweise 18 beziehungsweise 30 Stunden. Das Spülen kann in dem erfindungsgemäßen Verfahren sowohl in abwärts gerichteter Richtung (down-flow) als auch in aufwärts gerichteter Richtung (up-flow) durchgeführt werden.

Ein weiterer Gegenstand der vorliegenden Erfindung ist ein integriertes Verfahren zum Hydrieren eines aromatischen Wasserstoffes in Gegenwart eines Rutheniumkatalysators aufweisend einen Katalysator-Regenerierungsschritt. Dabei wird zuerst ein aromatischer Kohlenwasserstoff oder ein Gemisch von aromatischen Kohlenwasserstoffen, jeweils enthaltend schwefelhaltige aromatische Verbindungen, entsprechend dem erfindungsgemäßen Verfahren in Schritt a) entschwefelt und dann in Schritt b) hydriert. Anschließend wird in einem Regenerierungsschritt, wie vorstehend beschrieben, der Hydrierkatalysator mit Inertgas regeneriert, bis zum Erreichen der ursprünglichen oder eines Teils der ursprünglichen Aktivität.

Der aromatische Kohlenwasserstoff ist vorzugsweise Benzol. In einer bevorzugten Ausführungsform ist der Kohlenwasserstoff ein Gemisch aus Benzol und Toluol, oder ein Gemisch aus Benzol und Xylol bzw. einem Xylol-Isomerengemisch, oder ein Gemisch aus Benzol, Toluol und Xylol bzw. einem Xylol-Isomerengemisch.

Das erfindungsgemäße Verfahren eignet sich weiterhin zur Trocknung von Katalysatoren, die während verschiedener Vorgänge wie Wartung oder Lagerung Wasser aufgenommen haben.

Im Nachfolgenden soll anhand der aufgeführten Beispiele die Erfindung erläutert wer-, den:

### Beispiele für die Entschwefelung des aromatischen Kohlenwasserstoffes, oder des Gemisches von aromatischen Kohlenwasserstoffen (Stufe a)

Die Durchführung der Versuche erfolgte in kontinuierlich betriebenen Rohrreaktoren mit innen liegendem Thermoelementen (Ø 6 mm), Begleitheizung (Heizmatten) und Flüssigkeitsdosierung.

Als Entschwefelungsmittel wurde der Katalysator R 3-12 von BASF Aktiengesellschaft in Form von 5 x 3 mm Tabletten eingesetzt - nachfolgend Katalysator A genannt.

Die Trocknung des Entschwefelungmittels erfolgte gemäß der voranstehenden Beschreibung. Hierzu wurde das Entschwefelungsmittel in einem Stickstoffstrom von 300 ± 20 Nm³/m³ _{[KAT]}•h auf 200 ± 10°C erwärmt, wobei die Heizrate von 50 K/h nicht überschritten wurde. Sobald das Wasser entfernt war, wurde auf 120 ± 5°C, abgekühlt, wobei die Kühlrate von 50 K/h nicht überschreiten wurde. Das Trocknungsprocedere erfolgte in Rieselfahrweise (Strömungsrichtung von oben nach unten).

In einigen Fällen wurde das Entschwefelungsmittel in seiner reduzierten Form eingesetzt. Dabei wurde das Entschwefelungsmittel mit Wasserstoff gemäß der Beschreibung von seiner oxidierten Form in seine reduzierte Form übergeführt. Hierzu wurde das getrocknete Entschwefelungsmittel (in seiner oxidierten Form) auf 120 ± 5°C bei einer Stickstoffstrom von 300 ± 20 Nm³/m³ _{[KAT]}•h, erwärmt. Dann wurde zu dem oben genannten Stickstoffstrom 0,5 ± 0,1 Vol.-% Wasserstoff dosiert bis sich eine Temperaturerhöhung von 15 bis 20°C ergab und diese konstant blieb. Anschließend wurde der Wasserstoffstrom auf 1,0 ± 0,1 Vol.-% Wasserstoff erhöht bis sich insgesamt eine Temperaturerhöhung von max. 30 ± 5°C ergab und die Temperatur wiederum konstant blieb. Der Wasserstoffstrom wurde dann auf 2,0 ± 0,2 Vol.-% erhöht, wobei die Temperatur des Katalysators nicht über 225°C stieg. Der Wasserstoffstrom wurde dann auf 4,0 ± 0,4 Vol.-% erhöht und gleichzeitig die Temperatur des Stickstoffes auf 200 ± 10° erhöht, wobei hier die Temperatur des Katalysators nicht über 225°C stieg. Eine weitere Erhöhung des Wasserstoffstroms auf dann 6,0 ± 0,6 Vol.-% führte zu einem Anstieg der Temperatur des Katalysators auf 220 ± 10°C, die gehalten wurde. Nach einer Stunde wurde dann mit einem Stickstoffstrom von 300 ± 2 Nm³/ m³_{[KAT]}•h, auf unter 50°C abgekühlt, wobei die Kühlrate von 50 ± 5 K/h nicht überschreiten wurde. Das Reduktionsprocedere erfolgte in gerieselter Fahrweise (Strömungsrichtung von oben nach unten).

Als Einsatzstoff diente Benzol mit einer Reinheit von >99,95 %.

Die Analyse des eingesetzten Benzols und der Reaktionsausträge erfolgte durch Gaschromatographie unter Angabe von GC-Flächen % (Gerät: HP 5890-2 mit Probensampler; Range: 4; Säule: 30 m DB1; Filmdicke: 1 µm; Säuleninnendurchmesser: 0,25 mm; Probenvolumen: 5 µL; Trägergas: Helium; Flussrate: 100 mL/min; Injektortemperatur: 200°C; Detektor: FID; Detektortemperatur: 250°C; Temperaturprogramm: 6 min bei 40°C, 10 C/min bis 200 C für 8 min, Gesamtlaufzeit 30 min).

Die Analyse des Gesamtschwefegehalts im eingesetzten Benzol sowie von den Reaktionsausträgen erfolgte nach Wickbold-Verbrennung durch Ionenchromatographie. Hierzu werden 4 bis 6 g der Probe im Verhältnis 1:1 mit Aceton (Merck Suprasolv Artikel-Nr. 1.0012.1000) vermischt und danach in einer Verbrennungsapparartur nach Wickbold ("Wickbold Combustion") in der Knallgasflamme verbrannt. Das Verbrennungskondensat wird in einer alkalischen Vorlage aufgefangen, welche 40 mmol KOH (Merck Suprapure, Artikel-Nr 1.050.020.500) (wässrige Lösung) enthält. Das aus dem Schwefel gebildete und in der Vorlage aufgefangene Sulfat wird per lonenchromatographie bestimmt.
(Ionenchromatographie-System: modulares System, Fa. Metrohm; Vorsäule: DIONEX AG 12, 4mm; Trennsäule: DIONEX AS 12, 4 mm; Eluent: 2,7 mM Na₂CO₃ (Merck Suprapure, Artikel-Nr.1.063.950.500) und 0,28 mM NaHCO₃ (Riedel de Haen, p.A. Artikel-Nr. 31437); Fluss: 1 mL/min; Detektion: Leitfähigkeit nach chemischer Suppression; Suppressor: z.B. MSM, Fa. Metrohm).

### Beispiel a1

100 ml des Katalysators A, der gemäß des voranstehend geschilderten Trocknungsprocedere getrocknet wurde, wurden in oxidischer Form, in den oben beschriebenen Rohrreaktor (Ø 25 mm x 40 cm) gefüllt, wobei der Katalysator in eine Inertschüttung aus V4A-Ringen ober- und unterhalb der eigentlichen Katalysatorschüttung eingebettet wurde. Die Höhe der eigentlichen Katalysatorschüttung betrug ca. 22 cm. Der Versuch wurde in gesumpfter Fahrweise bei einem Druck von 20 bar durchgeführt, wobei während des Versuches 30 Nl Stickstoff pro h im Gleichstrom zum Flüssigkeitsstrom zudosiert wurden.

**Tabelle 1**

| Laufzeit | Temperatur | Katalysatorbelastung | Zulauf Benzol | | Austrag Gesamtschwefel | Austrag Benzol |
|---|---|---|---|---|---|---|
| H | °C | g/(ml•h) | g/h | Gesamtschwefel mg/kg | mg/kg | GC-FI.% |
| 187 | 140 | 0,50 | 50 | 0,4 | <0,1 | 99,9723 |
| 211 | 140 | 0,50 | 50 | 0,4 | <0,1 | 99,9733 |
| 220 | 140 | 2,00 | 200 | 0,4 | <0,1 | 99,9758 |
| 235 | 140 | 2,00 | 200 | 0,4 | <0,1 | 9,9755 |
| 245 | 140 | 2,00 | 200 | 0,4 | <0,1 | 99,9712 |
| 259 | 140 | 2,00 | 200 | 0,4 | <0,1 | 99,9671 |
| 355 | 120 | 2,00 | 200 | 0,4 | <0,1 | 99,9635 |
| 379 | 120 | 2,00 | 200 | 0,4 | <0,1 | 9,9646 |
| 386 | 120 | 2,00 | 200 | 0,4 | <0,1 | 9,9718 |
| 403 | 120 | 2,00 | 200 | 0,4 | <0,1 | 9,9756 |
| 427 | 120 | 2,00 | 200 | 0,4 | <0,1 | 99,9772 |
| 499 | 120 | 2,00 | 200 | 0,4 | <0,1 | 39,9742 |
| 523 | 120 | 2,00 | 200 | 0,4 | <0,1 | 99,9771 |
| 547 | 100 | 2,00 | 200 | 0,4 | <0,1 | 99,9772 |

Die in Tabelle 1 zusammengefassten Daten zeigen deutlich, dass die Entschwefelung des eingesetzten Benzols mit dem Katalysator A - in oxidischer Form - durchgeführt werden kann.

### Beispiel a2

100 ml des Katalysators A, der gemäß des voranstehend geschilderten Trocknungsprocedere getrocknet und der gemäß des voranstehend geschilderten Aktivierungsprocedere reduziert wurde, wurden in reduzierter Form, in den oben beschriebenen Rohrreaktor (Ø 25 mm x 80 cm) gefüllt, wobei der Katalysator in eine Inertschüttung aus V4A-Ringen ober- und unterhalb der eigentlichen Katalysatorschüttung eingebettet wurde. Die Höhe der eigentlichen Katalysatorschüttung betrug ca. 22 cm. Der Versuch wurde in gesumpfter Fahrweise bei einem Druck von 20 bar durchgeführt, wobei während des Versuches ein Gemisch von Stickstoff und Wasserstoff im Gleichstrom zum Flüssigkeitsstrom zudosiert wurden.

**Tabelle 2**

| Laufzeit | Temperatur | Zulauf | | Katalysatorbelastung | Zulauf Benzol | | Austrag Gesamtschwefel | Austrag Benzol |
|---|---|---|---|---|---|---|---|---|
| | | N₂ | H₂ | | | Gesamtschwefel | | |
| H | °C | Nl/h | Nl/h | g/(ml•h) | g/h | mg/kg | mg/kg | GC-FI.% |
| 187 | 80 | 30 | 2 | 2,04 | 204 | 0,4 | <0,1 | 99,9575 |
| 197 | 80 | 30 | 2 | 2,04 | 204 | 0,4 | <0,1 | 99,9564 |
| 211 | 30 | 30 | 2 | 2,04 | 204 | 0,4 | <0,1 | 99,9587 |
| 307 | 80 | 30 | 2 | 0,35 | 35 | 0,4 | <0,1 | 99,9502 |
| 331 | 30 | 30 | 2 | 2,04 | 204 | 0,4 | <0,1 | 99,9547 |
| 341 | 30 | 30 | 2 | 2,04 | 204 | 0,4 | <0,1 | 99,9572 |
| 379 | 40 | 30 | 2 | 2,04 | 204 | 0,4 | <0,1 | 99,9698 |
| 451 | 40 | 8 | 2 | 0,51 | 51 | 0,4 | <0,1 | 9,99657 |
| 475 | 40 | 8 | 2 | 2,04 | 204 | 0,4 | <0,1 | 99,9723 |
| 499 | 40 | 0 | 2 | 2,04 | 204 | 0,4 | <0,1 | 99,9730 |
| 548 | 40 | 0 | 2 | 0,30 | 30 | 0,4 | <0,1 | 99,9702 |
| 595 | 40 | 0 | 2 | 0,30 | 30 | 0,4 | <0,1 | 99,9663 |

Die in Tabelle 2 zusammengefassten Daten zeigen deutlich, dass die Entschwefelung des eingesetzten Benzols mit dem Katalysator A - in reduzierter Form - durchgeführt werden kann.

### Beispiel a3

100 ml des Katalysators A, der gemäß des voranstehend geschilderten Trocknungsprocedere getrocknet und der gemäß des voranstehend geschilderten Aktivierungsprocedere reduziert wurde, wurden in reduzierter Form, in den oben beschriebenen Rohrreaktor (0 25 mm x 40 cm) gefüllt, wobei der Katalysator in eine Inertschüttung aus V4A-Ringen ober- und unterhalb der eigentlichen Katalysatorschüttung eingebettet wurde. Die Höhe der eigentlichen Katalysatorschüttung betrug ca. 22 cm. Der Versuch wurde in gesumpfter Fahrweise bei einem Druck von 20 bar durchgeführt, wobei während des Versuches 2 Nl Wasserstoff pro h im Gleichstrom zum Flüssigkeitsstrom zudosiert wurden.

**Tabelle 3**

| Laufzeit | Temperatur | Katalysator belastung | Zulauf Benzol | | Austrag Gesamtschwefel | Austrag Benzol | Austrag Cyclohexan |
|---|---|---|---|---|---|---|---|
| H | °C | g/(ml_{Kat.} •h) | g/h | Gesamtschwefel mg/kg | mg/kg | GC-FI.% | GC-FI.% |
| 188 | 60 | 1,00 | 100 | 0,16 | <0, 1 | 99,9445 | 0,0360 |
| 196 | 80 | 1,00 | 100 | 0,16 | <<0,1 | 99,9372 | 0,0424 |
| 284 | 80 | 1,00 | 100 | 0,16 | <0,1 | 99,9369 | 0,0403 |
| 292 | 40 | 1,00 | 100 | 0,16 | <0,1 | 99,9372 | 0,0389 |
| 390 | 40 | 1,00 | 100 | 0,16 | <0,1 | 99,9641 | 0,0190 |
| 406 | 80 | 1,00 | 100 | 0,16 | <0,1 | 99,9528 | 0,0271 |
| 526 | 120 | 1,00 | 100 | 0,16 | <0,1 | 99,9421 | 0,0369 |
| 622 | 120 | 2,00 | 200 | 0,16 | <0,1 | 9,9429 | 0,0361 |
| 870 | 80 | 2,00 | 200 | 0,16 | <0,1 | 99,9677 | 0,0134 |
| 886 | 80 | 2,00 | 200 | 0,38 | <0,1 | 99,9686 | 0,0137 |
| 934 | 80 | 2,00 | 200 | 0,38 | <0,1 | 99,9682 | 0,0130 |
| 1222 | 80 | 2,00 | 200 | 0,45 | <0,1 | 99,9688 | 0,0129 |
| 1270 | 80 | 2,00 | 200 | 0,45 | <0,1 | 99,9700 | 0,0129 |
| 1294 | 80 | 2,00 | 200 | 0,45 | <0,1 | 99,9687 | 0,0147 |
| 1038 | 80 | 2,00 | 200 | 0,41 | <0,1 | 99,9678 | 0,0139 |
| 1126 | 80 | 2,00 | 200 | 0,41 | <0,1 | 99,9695 | 0,0122 |
| 1414 | 80 | 2,00 | 200 | 0,41 | 0,10 | 99,9666 | 0,0172 |
| 1462 | 80 | 2,00 | 200 | 0,56 | 0,12 | 99,9696 | 0,0139 |
| 1558 | 100 | 2,00 | 200 | 0,56 | <0,1 | 99,9686 | 0,014 |
| 1562 | 100 | 2,00 | 200 | 0,56 | <0,1 | 99,9682 | 0,0147 |

Die in Tabelle 3 zusammengefassten Daten zeigen deutlich, dass die Entschwefelung des eingesetzten Benzols mit dem Katalysator A - in reduzierter Form - auch im Dauerbetrieb durchgeführt werden kann. Weiterhin zeigen die Daten deutlich, dass nur geringste Mengen an Benzol zu Cyclohexan reduziert werden.

Nach Beendigung dieses Dauerversuches wurde der gebrauchte Katalysator ausgebaut und analysiert. Dazu wurde der Kontakt bei einer Temperatur von ca. 25-30 °C langsam mit einem Stickstoff/Luft-Gemisch bzw. mit reiner Luft oxidiert. Der oxidierte Katalysator wurde in zehn getrennten Fraktionen mit annähernd gleichen Volumina ausgebaut, jeweils eine Probe entnommen und diese per Elementaranalyse untersucht. Das Ergebnis der Analyse ist in Tabelle 4 aufgeführt. Die Proben sind entsprechend der Flussrichtung (gesumpfte Fahrweise, Fraktion 1 unten, Fraktion 10 oben) nummeriert.

**Tabelle 4**

| | Schwefel [ppm] |
|---|---|
| Ungebrauchter Katalysator A | 6 |
| Fraktion 10 | 250 |
| Fraktion 9 | 310 |
| Fraktion 8 | 300 |
| Fraktion 7 | 430 |
| Fraktion 6 | 440 |
| Fraktion 5 | 500 |
| Fraktion 4 | 870 |
| Fraktion 3 | 1100 |
| Fraktion 2 | 1400 |
| Fraktion 1 | 2400 |

Erwartungsgemäß weist die Katalysatorfraktion am Reaktoreingang (Fraktion 1) die höchste Schwefelkonzentration auf, während in der letzten Fraktion (Fraktion 10) der geringste Gehalt enthalten ist.

### Beispiel a4

50 ml des Katalysators A, der gemäß des voranstehend geschilderten Trocknungsprocedere getrocknet und der gemäß des voranstehend geschilderten Aktivierungsprocedere reduziert wurde, wurden in reduzierter Form, in den oben beschriebenen Rohrreaktor (∅ 25 mm x 40 cm) gefüllt, wobei der Katalysator in eine Inertschüttung aus V4A-Ringen ober- und unterhalb der eigentlichen Katalysatorschüttung eingebettet wurde. Die Höhe der eigentlichen Katalysatorschüttung betrug ca. 11 cm. Der Versuch wurde in gesumpfter Fahrweise bei einem Druck von 3 bar durchgeführt, wobei während des Versuches 2 NI Wasserstoff pro h im Gleichstrom zum Flüssigkeitsstrom zudosiert wurden.

**Tabelle 5**

| Laufzeit | Temperatur | Katalysatorbelastung | Zulauf Benzol | | Austrag | Austrag | Austrag |
|---|---|---|---|---|---|---|---|
| H | °C | g/(ml•h) | g/h | Gesamtschwefel mg/kg | Gesamtschwefel mg/kg | Benzol GC-FI.% | Cyclohexan GC-FI.% |
| 198 | 80 | 5,60 | 280 | 0,4 | <0,1 | 99,9768 | 0,0089 |
| 222 | 80 | 5,60 | 280 | 0,38 | <0,1 | 99,9792 | 0,0093 |
| 294 | 80 | 1,80 | 90 | 0,17 | <0,1 | 99,9783 | 0,0095 |
| 318 | 80 | 5,60 | 280 | 0,17 | <0,1 | 99,9779 | 0,0103 |
| 342 | 80 | 5,60 | 280 | 0,17 | <0,1 | 99,9803 | 0,0080 |
| 390 | 80 | 5,60 | 280 | 0,17 | <0,1 | 99,9776 | 0,0108 |
| 486 | 80 | 5,60 | 280 | 0,17 | <0,1 | 99,9789 | 0,009 |
| 510 | 80 | 5,60 | 280 | 0,19 | <0,1 | 99,9766 | 0,0108 |
| 654 | 80 | 1,10 | 55 | 0,19 | <0,1 | 99,9754 | 0,0117 |
| 678 | 80 | 5,60 | 280 | 0,19 | <0,1 | 99,9786 | 0,0093 |
| 702 | 80 | 5,60 | 280 | 0,18 | <0,1 | 99,9781 | 0,0099 |
| 726 | 80 | 5,60 | 280 | 0,18 | <0,1 | 99,9812 | 0,0086 |
| 798 | 80 | 1,80 | 90 | 0,18 | 0,1 | 99,9811 | 0,0084 |
| 846 | 80 | 5,60 | 280 | 0,18 | <0,1 | 99,9822 | 0,0075 |
| 870 | 80 | 5,60 | 280 | 0,18 | <0,1 | 99,9819 | 0,0075 |
| 894 | 80 | 5,60 | 280 | 0,14 | 0,1 | 99,9786 | 0,0109 |
| 966 | 80 | 1,80 | 90 | 0,1 | <0,1 | 99,9813 | 0,0073 |
| 990 | 80 | 5,60 | 280 | 0,1 | <0,1 | 99,982 | 0,0061 |
| 1014 | 80 | 5,60 | 280 | 0,1 | <0,1 | 99,9821 | 0,0061 |
| 1038 | 80 | 5,60 | 280 | 0,1 | <0,1 | 99,9803 | 0,0089 |
| 1062 | 80 | 5,60 | 280 | 0,1 | 0,1 | 99,9772 | 0,0117 |

Die in Tabelle 5 zusammengefassten Daten zeigen deutlich, dass eine Entschwefelung bei 3 bar, 80°C und Katalysatorbelastungen von >5 kg _{Benzol}/I_{Katalysator}•h durchgeführt werden kann.

### Beispiel a5

In einem kontinuierlich betriebenen Rohrreaktor (0 46 mm x 3500 mm) wurden 3700 ml des Katalysators A gefüllt, wobei der Katalysator in eine Inertschüttung ober- und unterhalb der eigentlichen Katalysatorschüttung eingebettet wurde (800 bzw. 500 ml). Der eingebaute Katalysator A wurde dann in Rieselfahrweise gemäß des in Tabelle 6 geschilderten Procedere getrocknet und reduziert.

**Tabelle 6**

| Zeit | Temperatur | | | | Zuläufe | | |
|---|---|---|---|---|---|---|---|
| | Katalysatorbett unten | Katalysatorbett mitte | Katalysatorbett oben | Vorheizer | H₂ | N₂ | |
| h | °C | °C | °C | °C | NI/h | NI/h | |
| 0 | 152 | 153 | 153 | 156 | 0 | 1500 | |
| 8 | 193 | 193 | 194 | 199 | 0 | 1500 | Trocknung |
| 11 | 203 | 203 | 203 | 207 | 0 | 1500 | |
| 28 | 201 | 201 | 201 | 205 | 0 | 1500 | |
| 33 | 102 | 102 | 104 | 104 | 15 | 1500 | Aktivierung (Reduktion) |
| 36 | 139 | 138 | 140 | 148 | 15 | 1500 | |
| 40 | 149 | 149 | 150 | 150 | 15 | 1500 | |
| 44 | 149 | 149 | 149 | 149 | 30 | 1500 | |
| 48 | 149 | 154 | 149 | 151 | 0 | 1500 | |
| 52 | 150 | 149 | 150 | 150 | 0 | 1500 | |
| 64 | 197 | 197 | 197 | 200 | 60 | 1500 | |
| 95 | 215 | 215 | 215 | 218 | 90 | 1500 | |
| 31 | 220 | 220 | 220 | 221 | 90 | 1500 | |
| 92 | 193 | 193 | 193 | 196 | 90 | 1500 | |
| 96 | 71 | 71 | 72 | 69 | 90 | 1500 | |
| 120 | 45 | 45 | 45 | 45 | 90 | 1500 | |

Anschließend wurde bei einem Druck von 3 bis 32 bar die Entschwefelung in gefluteter Fahrweise durchgeführt.

**Tabelle 7**

| Laufzeit | Temperatur | | | | | Zuläufe | | Katalysatorbelastung | Analytik Zulauf | | | | | Analytik Austrag Benzol | | | | |
|---|---|---|---|---|---|---|---|---|---|---|---|---|---|---|---|---|---|---|
| | | | | | Druck | | | | Schwefel-GC Benzol | | | | | Schwefel-GC | | | | |
| h | unten °C | mitte °C | oben °C | Vorheizer °C | bar | Benzol kg/h | H₂ /h | kg/(I*h) | COS ppb | Thiophen ppb | Gesamtschwefel mg/kg | GC Benzol Flächen-% | GC Cyclohexan Flächen-ppm | COS ppb | Thiophen ppb | Gesamtschwefel mg/kg | GC Benzol Flächen-% | GC Cyclohexan Flächen-ppm |
| 134 | 77 | 79 | 80 | 81 | 20 | 8,0 | 10 | 2,2 | | | 0,19 | 99,9613 | 156 | | | <0,1 | 99,8970 | 170 |
| 296 | 78 | 79 | 80 | 82 | 25 | 8,0 | 5 | 2,1 | | | 0,34 | -99,974 | 103 | | | <0,1 | 99,9651 | 182 |
| 440 | 77 | 79 | 80 | 82 | 28 | 8,0 | 17 | 2,2 | | | 0,20 | 99,9658 | 192 | | | <0,1 | 99,9510 | 213 |
| 512 | 77 | 79 | 80 | 82 | 31 | 8,0 | 25 | 2,2 | | | 0,18 | | | | | <0,1 | 99,9545 | 252 |
| 752 | 78 | 79 | 80 | 82 | 32 | 8,0 | 30 | 2,2 | | | 0,20 | | | | | <0,1 | 99,9442 | 353 |
| 972 | 77 | 79 | 80 | 82 | 32 | 8,0 | 29 | 2,2 | | | 0,2 | 99, 9522 | 273 | | | <0,1 | 99,952 | 273 |
| 944 | 77 | 79 | 80 | 82 | 32 | 8,0 | 30 | 2,2 | | | 0,20 | | | | | <0,1 | 99,9498 | 305 |
| 1016 | 77 | 79 | 80 | 82 | 32 | 8,0 | 30 | 2,2 | | | 0,27 | | | | | <0,1 | 99,9553 | 254 |
| 1912 | 77 | 79 | 80 | 82 | 32 | 8,0 | 30 | 2,2 | | | 0,28 | 99,9784 | 107 | <50 | <50 | <0,1 | 99,9385 | 489 |
| 1936 | 77 | 79 | 80 | 82 | 32 | 8,0 | 30 | 2,2 | | | 0,28 | | | <50 | <50 | 0,10 | 99,9598 | 276 |
| 1960 | 78 | 79 | 81 | 82 | 32 | 8,0 | 30 | 2,2 | | | 0,14 | 99,9772 | 123 | <50 | <50 | <0,1 | 99,9441 | 435 |
| 2104 | 78 | 79 | 81 | 82 | 32 | 8,0 | 30 | 2,2 | | | 0,19 | 99,9787 | 111 | <50 | <50 | <0,1 | 99,9434 | 467 |
| 2200 | 78 | 79 | 80 | 82 | 32 | 8,0 | 30 | 2,2 | 43 | 435 | 0, 20 | 99,986 | 47 | <50 | <50 | 0,13 | 99,9767 | 122 |
| 2296 | 77 | 79 | 80 | 83 | 32 | 8,0 | 30 | 2,2 | 35 | 227 | 0,47 | 99,9802 | 92 | <50 | <50 | <0,1 | 99,9741 | 149 |
| 2536 | 78 | 79 | 80 | 83 | 32 | 8,0 | 30 | 2,2 | 48 | 301 | 0,53 | 99,9812 | 77 | <50 | <50 | <0,1 | 99,9809 | 63 |
| 3232 | 77 | 79 | 80 | 82 | 32 | 8,0 | 30 | 2,2 | 20 | 295 | 0,28 | 99,9839 | 76 | <50 | <50 | <0,1 | 99,9829 | 83 |
| 3436 | 77 | 79 | 80 | 82 | 3 | 8,0 | 5 | 2,2 | 32 | 73 | 0,23 | 99,9807 | 106 | <50 | <50 | <0,1 | 99,9828 | 85 |
| 4036 | 77 | 79 | 80 | 82 | 3 | 8,0 | 5,5 | 2,2 | <50 | 75 | 0,24 | 99,9862 | 61 | <50 | <50 | <0,1 | 99,9835 | 57 |
| 4444 | 77 | 79 | 80 | 82 | 3 | 8,0 | 5,2 | 2,2 | <50 | 210 | 0,19 | 99,9785 | 107 | | | <0,1 | 99,9786 | 92 |
| 4940 | 76 | 78 | 79 | 81 | 3 | 8,0 | 6 | 2,2 | <50 | 440 | 0,15 | 99, 9744 | 103 | | | <0,1 | 99,9743 | 99 |
| 5892 | 77 | 78 | 79 | 81 | 3 | 8,0 | 7 | 2,2 | <50 | 190 | <0,1 | 99.976 | 48 | <50 | <50 | <0,1 | 99,9774 | 7 |
| 6780 | 76 | 78 | 79 | 81 | 3 | 8,0 | 4,4 | 2,2 | <50 | 390 | 0,24 | 99,9671 | | <50 | <50 | <0,1 | 99,9755 | 81 |
| 7188 | 76 | 78 | 80 | 82 | 3 | 8,0 | 5,3 | 2,2 | | | 0,14 | 99,9564 | 70 | | | <0,1 | 99,9605 | 88 |

Die Ergebnisse der Tabelle 7 zeigen deutlich, dass der Gehalt an schwefelhaltigen aromatischen Verbindungen unter 70 ppb abgesenkt werden kann.

### Beispiele für die Hydrierung des aromatischen Kohlenwasserstoffes, oder des Gemisches von aromatischen Kohlenwasserstoffen (Stufe b)

### Allgemeine Verfahrensbeschreibung 1 (AVB 1)

Die Versuchsdurchführung erfolgte in einem kontinuierlich betriebenen Doppelmantel-Reaktor (∅ 12 mm x1050 mm) mit drei auf die Reaktorlänge gleichmäßig verteilten Ölheizkreisen. Der Reaktor wurde mit mengengeregeltem Flüssigkeitsumlauf (HPLC-Pumpe) in kontinuierlicher Rieselfahrweise betrieben. Die Versuchanlage war ferner mit einem Abscheider zur Trennung von Gas- und Flüssigphase mit Standhaltung, Abgasregelung, externen Wärmetauscher und Probenahme ausgestattet. Die Dosierung des Wasserstoffs erfolgte druckgeregelt (in bar), die Messung des überschüssig eingesetzten Wasserstoffs erfolgte mengengeregelt (in NI/h), die Dosierung des Einsatzstoffes Benzol erfolgte über eine HPLC-Pumpe. Der Produktaustrag erfolgte standgeregelt über ein Ventil. Die Temperatur wurde am Anfang (Eingang) und am Ende (Ausgang) des Reaktors bzw. der Katalysatorschüttung mit einem Thermoelement gemessen. Das eingesetzte Benzol hatte einen Gesamtschwefelgehalt von <0,1 mg/kg (Nachweis durch Ionenchromatographie). Als Katalysator wurde ein meso-/makroporöser Ru/Al₂O₃-Katalysator, mit 0,47 Gew.-% Ru eingesetzt (Katalysator B) oder ein mesoporöser Ru/SiO₂-Katalysator mit 0,32 Gew.-% Ru (Katalysator C). Diese wurden wie in der Beschreibung aufgeführt hergestellt. Beispielsweise kann Katalysator C wie folgt hergestellt werden:
50 kg des SiO₂-Trägers (D11-10 (BASF); 3 mm Stränge (Nr. 04/19668), Wasseraufnahme von 0,95 ml/g, BET 135 m²/g) werden in einer Tränktrommel vorgelegt und bei 96-98 Gew.-% Wasseraufnahme getränkt. Die wässrige Tränklösung enthält 0,176 kg Ru als Ru-Acetat von Umicore, 4,34 Gew.-% Ru, Charge 0255). Der getränkte Katalysator wird unbewegt bei einer Ofentemperatur von 145°C getrocknet bis zu einer Restfeuchte von circa 1 %. Die Reduktion erfolgt in Wasserstoff bewegt (circa 75 % H₂ in N₂, wobei N₂ als Spülstrom angewendet wird; 1,5 Nm³/h H₂ - 0,5 Nm³/h N₂) bei bewegter Schüttung bei 300°C und einer Verweilzeit von 90 Minuten (1-2 h). Die Passivierung erfolgt in verdünnter Luft (Luft in N₂). Die Luftzugabe wird so geregelt, so dass die Temperatur des Katalysators unter 30-35°C bleibt. Der fertige Katalysator C enthält 0,31-0,32 Gew.-% Ru.

Im Folgenden ist dieser Katalysator näher beschrieben:

| | |
|---|---|
| Träger: | BASF-SiO₂-Träger D11-10 (3 mm Strängling) |
| Porosität des Formkörpers: | 0,95 ml/g |
| | (Wasseraufnahmebestimmung, indem der Träger mit Wasser gesättigt wird, und anschließend die überstehende Lösung und nach Abtropfen des Wassers die Menge aufgenommenes Wasser bestimmt wird. Dabei ist 1 ml Wasser = 1 g Wasser). |
| Rütteldichte des Formkörpers: | 467 g/l (bis Durchmesser des Formkörpers von 6 mm). |
| Bestimmung des Rutheniumgehalts: | 0,03 bis 0,05 Gramm der Probe wird in einem Alsint-Tiegel mit 5 g Natriumperoxid vermengt und auf einer Heizplatte langsam erhitzt. Anschließend wird das Substanz-Schmelzmittelgemisch zunächst über einer offenen Flamme geschmolzen und anschließend über einer Gebläseflamme bis zur Rotglut erhitzt. Der Aufschluss ist beendet, sobald eine klare Schmelze erreicht ist.Der abgekühlte Schmelzkuchen wird in 80 ml Wasser gelöst, die Lösung zum Sieden erhitzt (Zerstörung von H₂O₂) und anschließend - nach dem Abkühlen - mit 50 ml 21 Gew.% Salzsäure versetzt. Danach wird mit Wasser auf ein Volumen von 250 ml aufgefüllt. Die Messung dieser Probenlösung erfolgt per ICP-MS auf Isotop Ru 99. |
| Ru-Dispersität: | 90-95 % (nach CO-Sorption, angenommener Stöchiometrie-Faktor: 1; Probenpräparation: Reduktion der Probe für 30 min bei 200° C mit Wasserstoff und anschließend für 30 min mit Helium bei 200°C gespült - Messung der Metalloberfläche mit Pulsen von dem zu adsorbierenden Gas im Inertgasstrom (CO) bis zur Sättigung Chemisorption bei 35°C. Sättigung ist erreicht bis kein CO mehr adsorbiert wird, d.h. die Flächen von 3 bis 4 aufeinander folgenden Peaks (Detektorsignal) sind konstant und dem Peak eines nichtadsorbierten Pulses ähnlich. Pulsevolumen ist auf 1 % genau bestimmt, Druck und Temperatur des Gases muss überprüft werden). (Methode: DIN 66136) |
| Oberflächeanalyse - Porenverteilung | N₂-Sorption nach DIN 66131/DIN 66134 oder Hg-Porosimetrie nach DIN 66133) |
| | N₂-Sorption: BET 130-131 m²/g (DIN 66131) Mittlerer Porendurchmesser 26-27 nm (DIN 66134) Porenvolumen: 0,84-0,89 ml/g |
| | Hg Porosimetrie (DIN 66133): BET 119-122 m²/g Mittlerer Porendurchmesser (4V/A) 28-29 nm Porenvolumen: 0,86 -0,87 ml/g |

TEM:
Der reduzierte Katalysator C enthält zumindest teilweise kristallines Ruthenium in der äußersten Zone (Strangoberfläche). Im Träger kommt Ruthenium in Einzelteilchen vor 1-10 nm (> 5 nm stellenweise): meistens 1-5 nm. Die Größe der Partikeln nimmt von außen nach innen ab.

Ruthenium-Teilchen werden bis zu einer Tiefe von 30-50 Mikrometer unter der Strangoberfläche gesichtet. In dieser Schale liegt Ruthenium zumindest teilweise kristallin vor (SAD: selected area diffraction). Der Hauptteil des Rutheniums liegt also in dieser Schale (> 90 % innerhalb der ersten 50 µm).

### Allgemeine Versuchsbeschreibung 2 (AVB 2)

In einem beheizbaren 1,2 I-Druckbehälter (Innendurchmesser 90 mm, Behälterhöhe: 200 mm, aus Edelstahl mit 4-Blatt-Balkenbegasungsrührer, Stromstörern und einem innenliegender Steigleitung zur Probenahme bzw. zur Befüllung und Entleerung des Druckbehälters wird die jeweilige eingesetzte Menge (Volumen bzw. Masse) des Katalysators in einen "Katalysatorkorb" (aus Edelsstahl) gefüllt.

Der Druckbehälter wird zur Druckprüfung verschlossen und mit 50 bar Stickstoff beaufschlagt. Im Anschluss wird entspannt, der Druckbehälter mit einer Vakuumpumpe evakuiert, von der Vakuumpumpe getrennt und über die Steigleitung Einsatzstoff bzw. die Einsatzstofflösung in den Behälter eingesaugt.

Zur Entfernung von Restmengen an Sauerstoff wird bei Raumtemperatur der Behälter nacheinander zweimal mit je 10-15 bar Stickstoff und zweimal mit je 10-15 bar Wasserstoff beaufschlagt und entspannt.

Der Rührer wird eingeschaltet, eine Rührgeschwindigkeit von 1000 U/min eingestellt und die Reaktionslösung auf Reaktionstemperatur aufgeheizt. Die Solltemperatur ist nach spätestens 15 Minuten erreicht. Wasserstoff wird bis zu dem jeweiligen Solldruck innerhalb von 5 Minuten aufgepresst. Der Wasserstoffverbrauch wird ermittelt und der Druck konstant auf dem jeweiligen Solldruck gehalten.

Über die Steigleitung werden in regelmäßigen Abständen Vorproben (zur Spülung der Steigleitung) und Proben des Reaktionsansatzes zur Verfolgung des Reaktionsfortschritts entnommen.

Nach der entsprechenden Reaktionsdauer wird die Heizung ausgeschaltet, der Druckbehälter auf 25°C abgekühlt, der Überdruck langsam entspannt und der Reaktionsansatz mit leichtem Überdruck über die Steigleitung entleert. Anschließend wird der Druckbehälter mit der Vakuumpumpe evakuiert, der Druckbehälter von der Vakuumpumpe abgetrennt neuer Einsatzstoff bzw. die Einsatzstofflösung in den Behälter über die Steigleitung eingesaugt.

Dieses Vorgehen ermöglicht es den denselben Katalysator mehrmals einzusetzen. Der eingesetzte Wasserstoff hatte eine Reinheit von mindestens 99,9-99,99 Vol.% (bezogen auf trockenes Gas). Nebenbestandteile sind Kohlenmonoxid (max. 10 Vol.ppm), Stickstoff (max. 100 Vol.ppm), Argon (max. 100 Vol.ppm) und Wasser (max. 400 Vol.ppm).

Die Analyse des eingesetzten Benzols und der Reaktionsausträge erfolgte durch Gaschromatographie unter Angabe von GC-Flächen % (Gerät: HP 5890-2 mit Probensampler; Range: 4; Säule: 30 m DB1; Filmdicke: 1 µm; Säuleninnendurchmesser: 0,25 mm; Probenvolumen: 5 µL; Trägergas: Helium; Flussrate: 100 mL/min; Injektortemperatur: 200°C; Detektor: FID; Detektortemperatur: 250°C; Temperaturprogramm: 6 min bei 40 °C, 10 °C/min bis 200 °C für 8 min, Gesamtlaufzeit 30 min).

Die Analyse des Gesamtschwefegehalts im eingesetzten Benzol sowie von den Reaktionsausträgen erfolgte nach Wickbold-Verbrennung durch Ionenchromatographie. Hierzu werden 4 bis 6 g der Probe im Verhältnis 1:1 mit Aceton (Merck Suprasolv Artikel-Nr. 1.0012.1000) vermischt und danach in einer Verbrennungsapparartur nach Wickbold ("Wickbold Combustion") in der Knallgasflamme verbrannt. Das Verbrennungskondensat wird in einer alkalischen Vorlage aufgefangen, welche 40 mmol KOH (Merck Suprapure, Artikel-Nr 1.050.020.500) (in Wasser) enthält. Das aus dem Schwefel gebildete und in der Vorlage aufgefangene Sulfat wird per Ionenchromatographie bestimmt.
(Ionenchromatographie-System: modulares System, Fa. Metrohm; Vorsäule: DIONEX AG 12, 4mm; Trennsäule: DIONEX AS 12, 4. mm; Eluent: 2,7 mM Na₂CO₃ (Merck Suprapure, Artikel-Nr.1.063.950.500) und 0,28 mM NaHCO₃ (Riedel de Haen, p.A. Artikel-Nr. 31437); Fluss: 1 mL/min; Detektion: Leitfähigkeit nach chemischer Suppression; Suppressor: z.B. MSM, Fa. Metrohm).

### Beispiel b1 (nach AVB 1)

104 ml (63,9 g) des Katalysators B wurden zur kontinuierlichen Hydrierung bei einem Wasserstoffdruck von 32 bar, bei einem Abgas von 1-5 NI/h, einer Reaktoreingangstemperatur von 88-100°C und einem Zulauf-/Kreislaufyerhältnis von 1 : 30 eingesetzt. Die Ergebnisse sind in Tabelle 8 zusammengefasst.

### Beispiel b2 (nach AVB 1)

104 ml (63,9 g) des Katalysators B wurden zur kontinuierlichen Hydrierung bei einem Wasserstoffdruck von 19 bar, bei einem Abgas von 1-5 Nl/h, einer Reaktoreingangstemperatur von 88-100°C und einem Zulauf-/Kreislaufverhältnis von 1 : 30 eingesetzt. Die Ergebnisse sind in Tabelle 9 zusammengefasst.

### Beispiel b3 (nach AVB 1)

104 ml (45,0 g) des Katalysators C wurden zur kontinuierlichen Hydrierung bei einem Wasserstoffdruck von 32 bar, bei einem Abgas von 1-5 Nl/h, einer Reaktoreingangstemperatur von 88-100°C und einem Zulauf-/Kreislaufverhältnis von 1 : 30 eingesetzt. Die Ergebnisse sind in Tabelle 10 zusammengefasst.

**Tabelle 8 (^{a)} [GC-FI.%]; ^{b)} [GC-FI. ppm])**

| Druck | Laufzeit | Zulauf Benzol | Umlauf | Temperatur Reaktoreingang | Temperatur Reaktorausgang | C5-Alkane | n-Hexan | Methylcyclopentan | Benzol | Cyclohexan | Methylcyclohexan | Ethylcyclopentan | Toluol | Sonstige |
|---|---|---|---|---|---|---|---|---|---|---|---|---|---|---|
| [bar] | [h] | [g/h] | [g/h] | [°C] | [°C] | [GC-FI.ppm] | | | | [GC-FI.%] | [GC-FI.ppm] | | | |
| | Einsatzstoff | | | | | 9 | 0 | 14 | 99,9763 ^{a)} | 65 ^{b)} | 44 | 25 | 37 | 43 |
| 32 | 22 | 62 | 1860 | 92 | 130 | 20 | 134 | 34 | 303 | 99,9340 | 77 | 25 | 0 | 67 |
| 32 | 49 | 62 | 1860 | 90 | 129 | 23 | 163 | 33 | 296 | 99,9311 | 77 | 26 | 0 | 71 |
| 32 | 94 | 62 | 1860 | 90 | 129 | 20 | 172 | 35 | 187 | 99,9430 | 75 | 25 | 0 | 56 |
| 32 | 142 | 62 | 1860 | 90 | 129 | 20 | 171 | 35 | 239 | 99,9380 | 75 | 25 | 0 | 55 |
| 32 | 239 | 62 | 1860 | 90 | 129 | 21 | 173 | 34 | 292 | 99,9325 | 74 | 24 | 0 | 57 |
| 32 | 286 | 62 | 1860 | 90 | 129 | 20 | 174 | 34 | 322 | 99, 9291 | 77 | 25 | 0 | 57 |
| 32 | 404 | 62 | 1860 | 89 | 128 | 19 | 173 | 34 | 355 | 99, 9259 | 78 | 25 | 0 | 57 |
| 32 | 468 | 62 | 1860 | 90 | 128 | 22 | 201 | 36 | 258 | 99, 9326 | 77 | 25 | 0 | 55 |
| 20 | 540 | 62 | 1860 | 90 | 126 | 23 | 202 | 36 | 300 | 99,9282 | 76 | 25 | 0 | 56 |
| 20 | 588 | 62 | 1860 | 90 | 129 | 28 | 213 | 36 | 331 | 99,9235 | 77 | 25 | 0 | 55 |
| 20 | 698 | 62 | 1860 | 90 | 130 | 22 | 200 | 35 | 348 | 99, 9217 | 78 | 24 | 0 | 76 |

**Tabelle 9 (^{a)} [GC-FI.%]; ^{b)} [GC-FI. ppm])**

| Druck | Laufzeit | Zulauf Benzol | Umlauf | Temperatur Reaktoreingang | emperatur-Reaktorausgang | C5-Alkane | n-Hexan | Methylcyclopentan | Benzol | Cyclohexan | Methylcyclohexan | Ethylcyclopentan | Toluol | Sonstige |
|---|---|---|---|---|---|---|---|---|---|---|---|---|---|---|
| [bar] | [h] | [g/h] | [g/h] | [°C] | [°C] | [GC-FI.ppm] | | | | [GC-FI.%] | [GC-FI.ppm] | | | |
| Einsatzstoff | | | | | | 9 | 0 | 10 | 99,9526 ^{a)} | 150 ^{b)} | 115 | 63 | 35 | 92 |
| 19 | 3 | 62,5 | 1860 | 89 | 130 | 34 | 148 | 17 | 445 | 99,9060 | 133 | 46 | 0 | 134 |
| 19 | 16 | 62,5 | 1860 | 89 | 129 | 34 | 292 | 17 | 301 | 99,8975 | 152 | 65 | 0 | 181 |
| 19 | 24 | 53,6 | 1830 | 89 | 129 | 33 | 290 | 17 | 122 | 99,9204 | 153 | 65 | 0 | 133 |
| 19 | 40 | 53,6 | 1830 | 89 | 129 | 32 | 297 | 17 | 151 | 99,9169 | 151 | 65 | 0 | 135 |
| 19 | 48 | 53,6 | 1830 | 89 | 129 | 32 | 302 | 17 | 173 | 99,9147 | 151 | 64 | 0 | 131 |
| 19 | 64 | 53,6 | 1830 | 89 | 129 | 32 | 311 | 18 | 191 | 99,9126 | 149 | 64 | 0 | 127 |
| 19 | 72 | 53,6 | 1830 | 89 | 129 | 31 | 315 | 18 | 199 | 99,9118 | 149 | 63 | 0 | 125 |
| 19 | 122 | 53,6 | 1830 | 89 | 129 | 32 | 335 | 19 | 303 | 99, 8994 | 148 | 63 | 0 | 125 |
| 19 | 136 | 53,6 | 1830 | 89 | 129 | 33 | 335 | 19 | 316 | 99,8978 | 149 | 63 | 0 | 126 |
| 19 | 144 | 53,6 | 1830 | 89 | 129 | 33 | 339 | 19 | 337 | 99, 8950 | 150 | 64 | 0 | 127 |
| 19 | 160 | 53,6 | 1830 | 89 | 129 | 33 | 342 | 20 | 360 | 99,8925 | 149 | 63 | 0 | 128 |
| 19 | 164 | 53,6 | 1830 | 89 | 129 | 33 | 343 | 19 | 376 | 99,8907 | 151 | 64 | 0 | 126 |
| 19 | 168 | 53,6 | 1830 | 89 | 129 | 32 | 345 | 20 | 397 | 99, 8888 | 148 | 63 | 0 | 127 |

**Tabelle 10 (^{a)} [GC-FI.%]; ^{b)} [GC-FI. ppm])**

| Druck | Laufzeit | Zulauf Benzol | Umlauf | Temperatur Reaktoreingang | Temperatur Reaktorausgang | C5-Alkane | n-Hexan | Methylcyclopentan | Benzol | Cyclohexan | Methylcyclohexan | Ethylcyclopentan | Toluol | Sonstige |
|---|---|---|---|---|---|---|---|---|---|---|---|---|---|---|
| [bar] | [h] | [g/h] | [g/h] | [°C] | [°C] | [GC-FI.ppm] | | | | [GC-FI.%] | [GC-FI.ppm] | | | |
| Einsatzstoff | | | | | | 9 | 0 | 8 | 39,9728 ^{a)} | 0 ^{b)} | 55 | 29 | 135 | |
| 32 | 82 | 63 | 1860 | 90 | 128 | 35 | 213 | 19 | 0 | 99,9475 | 195 | 30 | 0 | 33 |
| 32 | 177 | 63 | 1860 | 90 | 128 | 30 | 196 | 17 | 0 | 99,9499 | 194 | 30 | 0 | 34 |
| 32 | 296 | 63 | 1860 | 89 | 128 | 29 | 185 | 18 | 0 | 99, 9512 | 195 | 30 | 0 | 31 |
| 32 | 416 | 63 | 1860 | 90 | 128 | 24 | 169 | 17 | 0 | 99,9526 | 196 | 31 | 0 | 37 |
| 32 | 512 | 63 | 1860 | 100 | 139 | 45 | 370 | 23 | 0 | 99,9299 | 197 | 31 | 0 | 35 |
| 32 | 680 | 63 | 1860 | 100 | 139 | 42 | 331 | 22 | 0 | 99,9344 | 194 | 30 | 0 | 37 |
| 32 | 802 | 63 | 1860 | 100 | 139 | 37 | 308 | 21 | 0 | 99,9375 | 195 | 31 | 0 | 33 |
| 32 | 921 | 63 | 1860 | 100 | 139 | 37 | 304 | 23 | 0 | 99,9371 | 197 | 31 | 0 | 37 |
| 32 | 993 | 63 | 1860 | 100 | 139 | 37 | 305 | 22 | 0 | 99,9438 | 144 | 24 | 0 | 30 |

Die in den Tabellen 8 bis 10 zusammengefassten Daten zeigen deutlich, dass Cyclohexan mit einer exzellenten Selektivität erhalten werden kann.

### Beispiel b4

Die Hydrieranlage besteht aus einem Lagertank für das entschwefelte Benzol, einem Vorlagebehälter, einer Dosierpumpe für Benzol, einem Hauptreaktor (Ø 45 x 2000 mm) mit Abscheider zur Trennung von Gas- und Flüssigkeit und Regelung zur Standhaltung, Flüssigkeitsumlauf mit Pumpe, und einem Wärmetauscher zur Abfuhr der gebildeten Reaktionswärme, einem Nachreaktor (Ø 22 mm x, L1500 mm) mit einem Abscheider zur Trennung von Gas- und Flüssigkeit und Regelung zur Standhaltung sowie einem Lagertank für den Hydrieraustrag . Der Haupt- und Nachreaktor waren je mit einem innenliegendem Thermoelement (Ø 6 mm im Hauptreaktor, Ø 3 mm im Nachreaktor) ausgerüstet. Beide Reaktoren wurden in gerieselter Fahrweise betrieben. Flüssigkeit und Gas wurden im Gleichstrom zudosiert.

Der Hauptreaktor wurde mit 2700 ml (1870 g), der Nachreaktor mit 340 ml (229 g) des Katalysators B befüllt. Der Hauptreaktor war zur Isolierung und Begleitheizung mit elektrischen Heizmatten versehen. Der Nachreaktor wurde für eine adiabate Betriebsweise gefertigt und war mit einer entsprechenden Isolierung versehen. Ober- und unterhalb des Katalysators wurde eine Inertschüttung eingefüllt (Maschendrahtringe ausEdelstahl).

Als Einsatzstoff diente entschwefeltes Benzol, das analog zu Beispiel a4 oder a5 hergestellt wurde, mit einem Gesamtschwefelgehalt von <0,1 mg/kg.

Die Analyse des Benzols und und des Cyclohexans erfolgte durch Gaschromatographie unter Angabe von GC-Flächen % bzw. GC-FI.-ppm; die Analysen wurde ohne internen Standard durchgeführt (Gerät: HP 5890-2 mit Probensampler; Range: 4; Säule: 30 m DB1; Filmdicke: 1 µm; Säuleninnendurchmesser: 0,25 mm; Probenvolumen: 5 µL; Trägergas: Helium; Flussrate: 100 mL/min; Injektortemperatur: 200°C; Detektor: FID; Detektortemperatur: 250°C; Temperaturprogramm: 6 min bei 40°C, 10 C/min bis 200°C für 8 min, Gesamtlaufzeit 30 min).

Die Analyse des Gesamtschwefegehalts im eingesetzten Benzol sowie von den Reaktionsausträgen erfolgte nach Wickbold-Verbrennung durch lonenchromatographie. Hierzu werden 4 bis 6 g der Probe im Verhältnis 1:1 mit Aceton (Merck Suprasolv Artikel-Nr. 1.0012.1000) vermischt und danach in einer Verbrennungsapparartur nach Wickbold ("Wickbold Combustion") in der Knallgasflamme verbrannt. Das Verbrennungskondensat wird in einer alkalischen Vorlage aufgefangen, welche 40 mmol KOH (Merck Suprapure, Artikel-Nr 1.050.020.500) (in Wasser) enthält. Das aus dem Schwefel gebildete und in der Vorlage aufgefangene Sulfat wird per Ionenchromatographie bestimmt.

(Ionenchromatographie-System: modulares System, Fa. Metrohm; Vorsäule: DIONEX AG 12, 4mm; Trennsäule: DIONEX AS 12, 4 mm; Eluent: 2,7 mM Na₂CO₃ (Merck Suprapure, Artikel-Nr.1.063.950.500) und 0,28 mM NaHCO₃ (Riedel de Haen, p.A. Artikel-Nr. 31437); Fluss: 1 mL/min; Detektion: Leitfähigkeit nach chemischer Suppression; Suppressor: z.B. MSM, Fa. Metrohm).

Teilweise wurden der Schwefelgehalt durch Gaschromatographie (Nachweisgrenzen je 50 ppb für COS und Thiophen) bestimmt (Trennsäule: CP SIL88 (100 % Cyanopropylpolysiloxan), Länge: 50 m; Filmdicke: 0,2 µm; Innendurchmesser: 0,25 mm; Trägergas: Helium; Vordruck: 1,5 bar; Split: on Column (ml/min); Septumspülung: 5 ml/min; Ofentemperatur: 60°C; Vorheizzeit: 10 min; Rate 1: 5°C/min; Ofentemperatur 1: 200°C; Nachheizzeit 1: 10 min; Rate 2: - ;Ofentemperatur 2: -; Nachheizzeit 2: -; Injektortemperatur: on Column (°C); Detektortemperatur: 220°C; Injektion: HP Autosampler; Injektionsvolumen: 1,0 µL; Detektortyp: PFPD ( Flammenphotometer); GC-Methode: Gew.%-Methode mit externer Kalibrierung; Besonderheiten: ON-Column Injektion und Spezialdetektor Flammenphotometer).

Zu Beginn wurde die Anlage bei 20 bar betrieben, der Anlagendruck wurde nach 860 Betriebsstunden auf 32 bar erhöht. Im nachgeschalteten Nachreaktor wurde bis zum Vollumsatz hydriert, im Reaktionsaustrag war praktisch kein Benzol mehr nachweisbar.

**Tabelle 11**

| Laufzeit | Druck | Hauptreaktor | | | | | | | Nachreaktor | | | |
|---|---|---|---|---|---|---|---|---|---|---|---|---|
| | | Temperatur Reaktoreingong | Temperazur Reaktorausgang | Zulauf Banzol | Umlauf | Abgas | Benzol | Cyclohexan | Temperatur Reaktoreingang | Temperatur Reaktorausgang | Benzol | Cyclohexan |
| [h] | [bar] | [°C] | [°C] | [g/h] | [kg/h] | [Nl/h] | [-FI.ppm] | [GC-FI. %] | [°C] | [°C] | [GG-FI.ppm | [GG-FI.%] |
| 18 | 20 | 82 | 120 | 750 | 22,5 | 20 | 0 | 99,9613 | 85 | 82 | 7 | 99,9616 |
| 41 | 20 | 90 | 122 | 750 | 22,5 | 20 | 0 | 99,9546 | 84 | 81 | 0 | 99,9556 |
| 65 | 20 | 90 | 122 | 750 | 22,5 | 20 | 0 | 99,9524 | 84 | 85 | 0 | 99,9523 |
| 693 | 20 | 90 | 123 | 1220 | 36,6 | 40 | 17 | 99,9461 | 88 | 88 | | |
| 613 | 20 | 90 | 124 | 1300 | 39,0 | 40 | 42 | 99,9468 | 91 | 88 | 0 | 99,9519 |
| 865 | 32 | 90 | 123 | 1300 | 39,0 | 40 | 63 | 99,9449 | 90 | 89 | 0 | 99,9515 |
| 884 | 32 | 90 | 124 | 1400 | 42,0 | 40 | 4 | 99,9627 | 92 | 89 | 0 | 99,9619 |
| 892 | 32 | 90 | 123 | 1400 | 42,0 | 40 | 0 | 99,9640 | 94 | 90 | 0 | 99,9836 |
| 1003 | 32 | 90 | 124 | 1620 | 45,6 | 40 | 58 | 99,9557 | 90 | 89 | 0 | 99.962 |
| 1099 | 32 | 90 | 124 | 1520 | 45,6 | 40 | 315 | 99,9286 | 90 | 69 | 0 | 99.6631 |
| 1337 | 32 | 90 | 124 | 1520 | 45,6 | 45 | 472 | 99,8979 | 90 | 89 | 0 | 99,9684 |
| 1502 | 32 | 90 | 125 | 1520 | 45,6 | 45 | 1555 | 99,6043 | 92 | 92 | 0 | 99,9702 |
| 1770 | 32 | 90 | 125 | 1620 | 48,6 | 45 | 2091 | 99,7552 | 90 | 91 | 0 | 99,9668 |
| 1996 | 92 | 90 | 125 | 1620 | 48,6 | 45 | 2659 | 99,7063 | 90 | 92 | 0 | 99,9702 |

Die vorliegenden Ergebnisse zeigen, dass das erfindungsgemäße Verfahren es ermöglicht Benzol vollständig umzusetzen und Cyclohexan in hohen Reinheiten zu erhalten.

### Beispiel b5

Der Versuch wurde unter den gleichen Bedingungen und der gleichen Anlage wie in Beispiel b4) beschrieben durchgeführt. Allerdings wurde der Hauptreaktor mit 2700 ml (1218 g) Katalysator C und der Nachreaktor mit 340 ml (153 g) Katalysator C befüllt. Weiterhin wurde die Anlage von Beginn an mit 32 bar betrieben.

Auch die hier erhaltenen Ergebnisse zeigen, dass im Reaktionsaustrag praktisch kein Benzol mehr nachweisbar ist.

Weiterhin wurde nach einer Laufzeit von 5347 h 4,3 Gew.-% Toluol in den Feed mit zudosiert. Es wurden im Reaktionsaustrag die entsprechenden Mengen an Methylcyclohexan gefunden, jedoch kein Toluol.

**Tabelle 12**

| Laufzeit | Hauptreaktor | | | | | | | Nachreaktor | | | | |
|---|---|---|---|---|---|---|---|---|---|---|---|---|
| | Temperatur-Reaktor eingang | Temperatur-Reaktorausgang | Zulauf Benzol | Umlauf | Belastung | Benzol | Cyclohexan | Temperatur-Reaktoreingang | Temperatur-Reaktorausgang | Benzol | Cyclohexan | Abgas |
| [h] | [°C] | [°C] | g/h | kg/h | kg_{Benzo}/(l*h) | [GC-FI. ppm] | [GC-FI.%] | [°C] | [°C] | [GC-FI. ppm] | [GC-FI.%] | [NI/h] |
| 12 | 84,8 | 97,1 | 810 | 48,6 | 0,3 | 0 | 99,9688 | 79,3 | 84,5 | 0 | 99,9689 | 80 |
| 36 | 85,0 | 97,6 | 810 | 48,6 | 0,3 | 0 | 99,9685 | 78,1 | 84,3 | 0 | 99,8679 | 80 |
| 204 | 95,0 | 116,4 | 1620 | 48,6 | 0,6 | 0 | 99,9649 | 86,9 | 87,6 | 0 | 99,9646 | 40 |
| 514 | 84,8 | 117,5 | 1620 | 48,6 | 0,6 | 0 | 99,9720 | 86,0 | 87,1 | 10 | 99.9717 | 40 |
| 1018 | 85,0 | 117,5 | 1620 | 48,6 | 0,6 | 0 | 99,9761 | 85,1 | 85,4 | 0 | 99,9751 | 40 |
| 1042 | 84,8 | 117,5 | 1620 | 48,6 | 0,6 | 0 | 99,9764 | 84,0 | 85,4 | 0 | 99,9749 | 40 |
| 1066 | 85,3 | 117,2 | 1620 | 48,6 | 0,6 | 0 | 99,9739 | 85,0 | 85,3 | 0 | 99,9748 | 40 |
| 1090 | 85,1 | 117,5 | 1620 | 48,6 | 0,6 | 0 | 99,9751 | 86,5 | 86,0 | 0 | 39,9751 | 40 |
| 1498 | 85,0 | 117,0 | 1620 | 48,6 | 0,6 | 0 | 99,9769 | 87,3 | 85,9 | 0 | 99,9769 | 20 |
| 2002 | 84,8 | 116,4 | 1620 | 48,6 | 0,6 | 0 | 99,9769 | 85,2 | 85,6 | 0 | 99,9767 | 20 |
| 2508 | 84,8 | 116,3 | 1620 | 48,6 | 0,6 | 0 | 99,9790 | 85,6 | 85,9 | 0 | 99,9790 | 20 |
| 3012 | 85,1 | 117,5 | 1620 | 48,6 | 0,6 | 0 | 99.9792 | 84,9 | 85,9 | 0 | 99,9789 | 20 |
| 3276 | 84,8 | 118,5 | 1620 | 45,6 | 0,6 | 0 | 99,9790 | 85,0 | 85,7 | 0 | 99,9787 | 20 |
| 3324 | 84,8 | 124,1 | 1620 | 38,9 | 0,6 | 0 | 99,9767 | 85,1 | 85,5 | 0 | 99,9763 | 20 |
| 3516 | 85,0 | 124,9 | 1620 | 38,9 | 0,6 | 0 | 99,9768 | 83,3 | 85,6 | 0 | 99,9767 | 20 |
| 4020 | 84,8 | 124,7 | 1620 | 38,9 | 0,6 | 0 | 99,9729 | 85,0 | 85,7 | 0 | 99,9727 | 20 |
| 4524 | 85,0 | 125,4 | 1620 | 38,9 | 0,6 | 0 | 99,9696 | 83,4 | 85,4 | 0 | 99,9694 | 20 |
| 5012 | 84,8 | 123,4 | 1620 | 38,9 | 0,6 | 0 | 99,9764 | 84,7 | 85,6 | 0 | 99,9767 | 20 |
| 5299 | 84,8 | 124,1 | 1620 | 38,9 | 0,6 | 0 | 99,9731 | 85,1 | 85,8 | 0 | 99,9729 | 20 |

### Beispiel b6 (nach AVB 2)

750 ml einer 5 %-igen Lösung von Benzol in Cyclohexan wurden mit 9,0 g (ca. 22 ml) Katalysator C bei einer Temperatur von 100°C und einem Druck von 20 bar mit Wasserstoff hydriert.

Der Katalysator wurde in fünf aufeinander folgenden Versuchen wiederholt eingesetzt. Probenahme erfolgt nach Reaktionszeiten von 10, 20, 30, 40, 60, 90, 120 und 180 Minuten.

In Tabelle 13 ist die Abnahme des Benzolgehalts über die Zeit aufgeführt. Es werden die Mittelwerte der Ergebnisse der fünf Versuche sowie die maximale Abweichung vom Mittelwert nach oben und nach unten der jeweiligen Proben ausgewertet, Die Bestimmung des Benzolgehaltes erfolgte über GC-Analytik in GC-Flächen-%

**Tabelle 13**

| Reaktionszeit [min] | Benzolgehalt (Mittelwert der 5 Versuche) [GC-FI.%] | Maximale Abweichung vom Mittelwert nach unten | Maximale Abweichung vom Mittelwert nach oben |
|---|---|---|---|
| 0 (Ausgangslösung) | 5,394 | -0,015 | +0,005 |
| 10 | 3,728 | -0,520 | +0,343 |
| 20 | 2,647 | -0,669 | +0,367 |
| 30 | 1,655 | -0,718 | +0,509 |
| 40 | 0,943 | -0,851 | +0,562 |
| 60 | 0,100 | -0,097 | +0,159 |
| 90 | 0,002 | -0,002 | +0,003 |
| 120 | 0 | 0 | 0 |
| 180 | 0 | 0 | 0 |

### Beispiel b7 (nach AVB 2)

750 ml einer 5 %-igen Lösung von Benzol in Cyclohexan wurden mit 9,0 g (ca. 22 ml) Katalysator C bei einer Temperatur von 100°C und einem Druck von 32 bar mit Wasserstoff hydriert.

Der Katalysator wurde in fünf aufeinander folgenden Versuchen wiederholt eingesetzt. Probenahme erfolgt nach Reaktionszeiten von 10, 20, 30, 40, 60, 90, 120 und 180 Minuten.

In Tabelle 14 ist die Abnahme des Benzolgehalts über die Zeit aufgeführt. Es werden die Mittelwerte der Ergebnisse der fünf Versuche sowie die maximale Abweichung vom Mittelwert nach oben und nach unten der jeweiligen Proben ausgewertet, Die Bestimmung des Benzolgehaltes erfolgte über GC-Analytik in GC-Flächen-%

**Tabelle 14**

| Reaktionszeit [min] | Benzolgehalt (Mittelwert der 5 Versuche) [GC-FI.%] | Maximale Abweichung vom Mittelwert nach unten | Maximale Abweichung vom Mittelwert nach oben |
|---|---|---|---|
| 0 (Ausgangslösung) | 5,394% | 0 | 0 |
| 10 | 3,005% | -0,529 | +1,074 |
| 20 | 1,263% | -0,713 | +1,176 |
| 30 | 0,399% | -0,321 | +0,503 |
| 40 | 0,080% | -0,072 | +0,164 |
| 60 | 0,002% | -0,001 | +0,001 |
| 90 | 0.001% | -0,000 | +0,001 |
| 120 | 0,001% | -0,001 | +0,001 |
| 180 | 0% | 0 | 0 |

### Beispiel c

### Regenerierung eines Hydrierkatalysators

### Herstellungsbeispiel des Rutheniumkatalysators

Ein meso-/makroporöser Aluminiumoxidträger in Form von 3 bis 5 mm-Kugeln mit einem Gesamtvolumen von 0,44 cm³/g, wobei 0,09 cm³/g (20% des Gesamtporenvolumens) von Poren mit einem Durchmesser im Bereich von 50 nm bis 10.000 nm und 0,35 cm³/g (80% des Gesamtporenvolumens) von Poren mit einem Durchmesser im Bereich von 2 nm bis 50 nm gebildet werden, einem mittleren Porendurchmesser im Bereich von 11 nm und einer Oberfläche von 286 m²/g wurde mit einer wässrigen Ruthenium-(III)-nitrat-Lösung getränkt. Das während des Tränkens aufgenommene Lösungsvolumen entsprach dabei in etwa dem Porenvolumen des verwendeten Trägers. Anschließend wurde der mir der Ruthenium-(III)-nitrat-Lösung getränkte Träger bei 120°C getrocknet und bei 200°C im Wasserstrom aktiviert (reduziert). Der so hergestellte Katalysator enthielt 0,5 Gew.-% Ruthenium, bezogen auf das Gewicht des Katalysators. Die Rutheniumoberfläche betrug 0,72 m²/g, das Verhältnis von Ruthenium- zu Trägeroberfläche lag bei 0,0027.

### Beispiel 1 Sorptionsuntersuchungen

Durch Sorptionsmessungen von Wasserdampf an dem wie vorstehend beschrieben hergestellten Katalysator (0,5 % Ru/γ-Al₂O₃), wurde die Affinität des Katalysators zum Wasser ermittelt.

Es zeigte sich, dass der Katalysator schon bei geringen relativen Dampfdrücken von 30% eine Wässermenge von 5% sorbiert. Falls im Reaktor beziehungsweise in den Einsatzstoffen Wasser auch nur in Spuren vorhanden ist, kann dieses Wasser am Katalysator sorbiert werden.

### Beispiel 2 Standzeitversuch bei der Hydrierung von Benzol

In einer Anlage zur Herstellung von Cyclohexan unter Verwendung eines Ruthenium/Aluminiumoxidkatalysators mit 0,5 % Ru auf einem Träger aus Y-Al₂O₃ wird im Produktstrom ein stetiges Nachlassen der Katalysatoraktivität und ein zunehmender Benzolgehalt beobachtet. Ein weiteres Verfolgen der Reaktion zeigt, dass während eines Katalysatorstandzeittests bei der Hydrierung von Benzol der Restbenzolgehalt nach dem Hauptreaktor innerhalb einer Laufzeit von ca. 3.400 h von wenigen Hundert ppm auf einige Tausend ppm ansteigt. Eine Berechnung ergibt, dass bei der Zuführung von 16.620 kg/h Benzol mit einem Wassergehalt von 30 bis 50 ppm, 0,8 kg Wasser pro Stunde in die Anlage eingebracht werden. Hinzu kommen noch weitere 3,5 kg/h Wasser, welche aus dem Wasserstoffgas stammen.

Bei Abschalten der Anlage bei 3.394 Betriebsstunden lief die Anlage mit einem Restbenzolgehalt von 0,2 % bei einer Belastung von 0,6 g_{Benzol}/ml_{Kat}·h. Während des Abschaltens wurde die Anlage bei einer Temperatur von 70 bis 100°C mit Stickstoff ausgepresst und dann entspannt. Nach dem Anfahren lieferte die Anlage einen Restbenzolgehalt von 0,01 % bis 0,04 % bei einer Belastung von 0,6 g_{Benzol}/ml_{Kat}·h.

Dieser beobachtete Effekt des Trocknens des Katalysators wurde nach 7.288 Betriebsstunden erneut verifiziert. Bei einer Belastung von 0,9 g_{Benzol}/ml_{Kat}·h lag der Restbenzolgehalt am Ende der Anlage bei 0,2% und stieg sogar auf 0,56% an. Nach dem Abstellen der Anlage wurde der Katalysator über einen Zeitraum von 34 h bei 110°C mit 100 Nl/h Stickstoff getrocknet. Nach dem Anlaufen der Anlage mit einer Belastung von 0,6 g_{Benzol}/ml_{Kat}·h lag der Restbenzolgehalt bei 0,03 % bis 0,07 %, was auf eine deutliche Steigerung der Katalysatoraktivität durch das Trocknen zurückgeführt werden kann.

In beiden Fällen führte die Trocknung des Katalysators zu einer signifikant höheren Katalysatoraktivität, die nahe oder gleich der ursprünglichen Katalysatoraktivität liegt.

### Beispiel 3 Untersuchung des Wassereinflusses auf die Benzolhydrierung

Zur Simulierung des Wassereinflusses auf die Hydrierung von Benzol mit einem Rutheniumkatalysator wurden Versuchsreihen in Autoklavenversuchen vor und nach der Sättigung des Katalysators mit Wasser sowie nach Trocknung des Katalysators durchgeführt. Im Druckbehälter wurden eine 5 %ige Lösung von Benzol in Cyclohexan mit dem Rutheniumkatalysator vorgelegt, auf die Reaktionstemperatur von 100 °C aufgeheizt und der Reaktionsverlauf bei 32 bar Wasserstoffdruck durch regelmäßige Probennahme verfolgt. Die Proben wurden im Anschluss durch Gaschromatographie untersucht.

Es wurden 23 Hydrierversuche durchgeführt, der Katalysator im Anschluss ins Wasser gestellt. Danach wurden 13 weitere Hydrierversuche durchgeführt. Der Katalysator zeigte eine deutlich geringere, aber nahezu konstante Aktivität. Nach Trocknung des Katalysators im Stickstoffstrom bei 100°C in einem Reaktionsrohr wurden 5 weitere Versuche durchgeführt; der Katalysator zeigte eine ähnliche Hydrieraktivität wie vor der Sättigung mit Wasser.

Die Versuche belegen, dass die Aktivität des eingesetzten Ruthenium/AluminiumoxidKatalysators nach Kontakt mit Wasser signifikant nachlässt, der Katalysator aber durch Trocknung im Stickstoffstrom wieder reaktiviert und die Anfangsaktivität nahezu vollständig wieder hergestellt werden kann.

## Patentansprüche

1. Verfahren zur Umsetzung eines aromatischen Kohlenwasserstoffes, oder eines Gemisches von aromatischen Kohlenwasserstoffen, der schwefelhaltige aromatische Verbindungen enthält, wobei in einem ersten Schritt ggf. in Gegenwart von Wasserstoff, der Gehalt an schwefelhaltigen aromatischen Verbindungen emiedrigt wird, indem ein Entschwefelungsmittel eingesetzt wird, das Kupfer und Zink in einem Atomverhältnis von 1 : 0,3 bis 1 : 10 enthält (Schritt a), und in einem zweiten Schritt der aromatische Kohlenwasserstoff, oder das Gemisch der aromatischen Kohlenwasserstoffe, in Gegenwart eines geträgerten Rutheniumkatalysators und in Gegenwart von Wasserstoff hydriert wird (Schritt b).

2. Verfahren gemäß Anspruch 1, wobei der Gehalt an schwefelhaltigen aromatischen Verbindungen auf ≤ 70 ppb, und der Gesamtschwefelgehalt auf insgesamt ≤ 200 ppb emiedrigt wird.

3. Verfahren gemäß den Ansprüchen 1 oder 2, wobei in Schritt a) ein Entschwefelungsmittel eingesetzt wird, das Kupfer und Zink in einem Atomverhältnis von 1 : 0,5 bis 1 : 3, insbesondere von 1 : 0,7 bis 1 : 1,15, enthält.

4. Verfahren nach den Ansprüchen 1 bis 3, wobei das Entschwefelungsmittel 35 bis 45 Gew.-% Kupferoxid, 35 bis 45 Gew.-% Zinkoxid, 10 bis 30 Gew.-% Aluminiumoxid enthält.

5. Verfahren nach den Ansprüchen 1 bis 4, wobei Stufe a) in Abwesenheit von Wasserstoff durchgeführt wird.

6. Verfahren nach den Ansprüchen 1 bis 4, wobei Stufe a) in Gegenwart von Wasserstoff durchgeführt wird.

7. Verfahren nach den Ansprüchen 1 bis 6, wobei das Entschwefelungsmittel in oxidierter Form in Stufe a) eingesetzt wird.

8. Verfahren nach den Ansprüchen 1 bis 6 wobei das Entschwefelungsmittel in reduzierter Form in Stufe a) eingesetzt wird.

9. Verfahren nach den Ansprüchen 1 bis 8, wobei Schritt a) bei einer Temperatur von 40 bis 200 °C und einem Druck von 1 bis 40 bar, vorzugsweise von 1 bis 32 bar, insbesondere von 1,5 bis 5 bar durchgeführt wird.

10. Verfahren nach den Ansprüchen 1 bis 9, wobei der in Schritt b) eingesetzte geträgerte Rutheniumkatalysator ein Gehalt an Ruthenium 0,01 bis 30 Gew.-% bezogen auf das Gesamtgewicht des Katalysators hat.

11. Verfahren nach den Ansprüchen 1 bis 10 wobei der in Schritt b) eingesetzte geträgerte Rutheniumkatalysator als Trägermaterial Aluminiumoxid enthält.

12. Verfahren nach den Ansprüchen 1 bis 10, wobei der in Schritt b) eingesetzte geträgerte Rutheniumkatalysator als Trägermaterial Siliciumoxid enthält.

13. Verfahren nach Anspruch12, wobei es sich bei dem geträgerte Rutheniumkatalysator um einen Schalenkatalysator handelt, wobei mindestens 60 Gew.-% des Aktivmetalls in der Schale des Katalysators bis zu einer Eindringtiefe von 200 µm vorliegen.

14. Verfahren nach den Ansprüchen 1 bis 13, wobei Schritt b) bei einer Temperatur von 50 bis 250 °C, vorzugsweise von 60 bis 200 °C, insbesondere von 70 bis 170 °C und einem Druck von 1 bis 200 bar, besonders von 10 bis 50 bar, vor zugsweise von 19 bis 40 bar, insbesondere von 25 bis 35 bar durchgeführt wird.

15. Verfahren nach den Ansprüchen 1 bis 14, wobei als aromatischer Kohlenwasserstoff Benzol eingesetzt wird.

16. Verfahren nach den Ansprüchen 1 bis 15, wobei in einem weiteren Schritt c) das erhaltene Reaktionsprodukt einer weiteren Reinigung unterworfen wird.

17. Verfahren nach den Ansprüchen 1 bis 16, wobei in einem weiteren Schritt c) das erhaltene Reaktionsprodukt destillativ aufgereinigt wird.

18. Verfahren nach den Ansprüchen 1 bis 6, 8 bis 11 und 14 bis 17, wobei Schritt a) bei einem Druck von 2 bis 4,5 bar, einer Temperatur von 50 bis 180 °C in Gegenwart eines Entschwefelungsmittel, das 35 bis 45 Gew.% Kupferoxid, 35 bis 45 Gew.% Zinkoxid, 10 bis 30 Gew.% Aluminiumoxid enthält, und das in reduzierter Form eingesetzt wird, durchgeführt wird, und Schritt b) bei einem Druck von 19 bis 40 bar, einer Temperatur von 70 bis 170 °C in Gegenwart eines auf Aluminiumoxid geträgerten Rutheniumkatalysators, dessen Gehalt an Ruthenium bei 0,01 bis 30 Gew.% bezogen auf das Gesamtgewicht des Katalysators liegt, durchgeführt wird.

19. Verfahren nach den Ansprüchen 1 bis 6, 8 bis 10 und 12 bis 17 wobei Schritt a) bei einem Druck von 2 bis 4,5 bar, einer Temperatur von 50 bis 180°C in Gegenwart eines Entschwefelungsmittel, das 35 bis 45-Gew.-% Kupferoxid, 35 bis 45-Gew.% Zinkoxid, 10 bis 30 Gew.% Aluminiumoxid enthält, und das in reduzierter Form eingesetzt wird, durchgeführt wird, und Schritt b) bei einem Druck von 19 bis 40 bar, einer Temperatur von 70 bis 170°C in Gegenwart eines auf siliciumoxidgeträgerten Rutheniumkatalysators, dessen Gehalt an Ruthenium bei 0,01 bis 30 Gew.% bezogen auf das Gesamtgewicht des Katalysators liegt, durchgeführt wird.

20. Verfahren nach den Ansprüchen 18 oder 19, wobei Schritt a) in Gegenwart von Wasserstoff durchgeführt wird.

21. Verfahren zur Hydrierung von aromatischen Kohlenwasserstoffen nach einem den Ansprüche 1-20, darüben hinaus aufweisend einen Katalysator Regenerierungsschritt, umfassend das Spülen des Katalysators mit Inertgas bis zum Erreichen der ursprünglichen oder eines Teils der ursprünglichen Aktivität.

## Claims

1. A process for converting an aromatic hydrocarbon or a mixture of aromatic hydrocarbons, each of which comprises aromatic sulfur compounds, wherein, in a first step, optionally in the presence of hydrogen, the content of aromatic sulfur compounds is lowered by using a desulfurizing agent comprising copper and zinc in an atomic ratio of from 1 : 0.3 to 1 : 10 (step a), and, in a second step, the aromatic hydrocarbon or the mixture of aromatic hydrocarbons is hydrogenated in the presence of a supported ruthenium catalyst and in the presence of hydrogen (step b).

2. The process according to claim 1, wherein the content of aromatic sulfur compounds is lowered to ≤ 70 ppb, and the total sulfur content to a total of ≤ 200 ppb.

3. The process according to claim 1 or 2, wherein a desulfurizing agent used in step a) comprises copper and zinc in an atomic ratio of from 1 : 0.5 to 1 : 3, in particular from 1 : 0.7 to 1 : 1.15.

4. The process according to claims 1 to 3, wherein the desulfurizing agent comprises from 35 to 45% by weight of copper oxide, from 35 to 45% by weight of zinc oxide, from 10 to 30% by weight of aluminum oxide.

5. The process according to claims 1 to 4, wherein stage a) is carried out in the absence of hydrogen.

6. The process according to claims 1 to 4, wherein stage a) is carried out in the presence of hydrogen.

7. The process according to claims 1 to 6, wherein the desulfurizing agent is used in stage a) in oxidized form.

8. The process according to claims 1 to 6, wherein the desulfurizing agent is used in stage a) in reduced form.

9. The process according to claims 1 to 8, wherein step a) is carried out at a temperature of from 40 to 200°C and a pressure of from 1 to 40 bar, preferably from 1 to 32 bar, in particular from 1.5 to 5 bar.

10. The process according to claims 1 to 9, wherein the supported ruthenium catalyst used in step b) has a content of ruthenium of from 0.01 to 30% by weight based on the total weight of the catalyst.

11. The process according to claims 1 to 10, wherein the supported ruthenium catalyst used in step b) comprises aluminum oxide as the support material.

12. The process according to claims 1 to 10, wherein the supported ruthenium catalyst used in step b) comprises silicon oxide as the support material.

13. The process according to claim 12, wherein the supported ruthenium catalyst is a coated catalyst, at least 60% by weight of the active metal in the coating of the catalyst being present up to a penetration depth of 200 µm.

14. The process according to claims 1 to 13, wherein step b) is carried out at a temperature of from 50 to 250°C, preferably from 60 to 200°C, in particular from 70 to 170°C, and a pressure of from 1 to 200 bar, particularly from 10 to 50 bar, preferably from 19 to 40 bar, in particular from 25 to 35 bar.

15. The process according to claims 1 to 14, wherein the aromatic hydrocarbon used is benzene.

16. The process according to claims 1 to 15, wherein the reaction product obtained is subjected to a further purification in a further step c).

17. The process according to claims 1 to 16, wherein the reaction product obtained is purified by distillation in a further step c).

18. The process according to claims 1 to 6, 8 to 11 and 14 to 17, wherein step a) is carried out at a pressure of from 2 to 4.5 bar, a temperature of from 50 to 180°C, in the presence of a desulfurizing agent which comprises from 35 to 45% by weight of copper oxide, from 35 to 45% by weight of zinc oxide, from 10 to 30% by weight of aluminum oxide, and which is used in reduced form, and step b) is carried out at a pressure of from 19 to 40 bar, a temperature of from 70 to 170°C, in the presence of a ruthenium catalyst supported on aluminum oxide, whose content of ruthenium is from 0.01 to 30% by weight based on the total weight of the catalyst.

19. The process according to claims 1 to 6, 8 to 10 and 12 to 17, wherein step a) is carried out at a pressure of from 2 to 4.5 bar, a temperature of from 50 to 180°C, in the presence of a desulfurizing agent which comprises from 35 to 45% by weight of copper oxide, from 35 to 45% by weight of zinc oxide, from 10 to 30% by weight of aluminum oxide, and which is used in reduced form, and step b) is carried out at a pressure of from 19 to 40 bar, a temperature of from 70 to 170°C, in the presence of a ruthenium catalyst supported on silicon oxide, whose content of ruthenium is from 0.01 to 30% by weight based on the total weight of the catalyst.

20. The process according to claim 18 or 19, wherein step a) is carried out in the presence of hydrogen.

21. A process for hydrogenating aromatic hydrocarbons according to any of claims 1 - 20, additionally comprising a catalyst regeneration step, comprising rinsing the catalyst with inert gas until attainment of the original or some of the original activity.

## Revendications

1. Procédé de transformation d'un hydrocarbure aromatique, ou d'un mélange d'hydrocarbures aromatiques, qui contient des composés aromatiques contenant du soufre, dans lequel, lors d'une première étape, la teneur en composés aromatiques contenant du soufre est réduite éventuellement en présence d'hydrogène en utilisant un agent de désulfuration qui contient du cuivre et du zinc en un rapport atomique de 1:0,3 à 1:10 (étape a) et, lors d'une seconde étape, l'hydrocarbure aromatique, ou le mélange d'hydrocarbures aromatiques, est hydrogéné en présence d'un catalyseur de ruthénium supporté ou en présence d'hydrogène (étape b).

2. Procédé selon la revendication 1, dans lequel la teneur en composés aromatiques contenant du soufre est réduite à ≤ 70 ppb, et la teneur en soufre totale est réduite au total à ≤ 200 ppb.

3. Procédé selon la revendication 1 ou 2, dans lequel, lors de l'étape a), un agent de désulfuration qui contient du cuivre et du zinc en un rapport atomique de 1:0,5 à 1:3, notamment de 1:0,7 à 1:1,15, est utilisé.

4. Procédé selon les revendications 1 à 3, dans lequel l'agent de désulfuration contient 35 à 45 % en poids d'oxyde de cuivre, 35 à 45 % en poids d'oxyde de zinc, 10 à 30 % en poids d'oxyde d'aluminium.

5. Procédé selon les revendications 1 à 4, dans lequel l'étape a) est réalisée en l'absence d'hydrogène.

6. Procédé selon les revendications 1 à 4, dans lequel l'étape a) est réalisée en la présence d'hydrogène.

7. Procédé selon les revendications 1 à 6, dans lequel l'agent de désulfuration est utilisé sous forme oxydée à l'étape a).

8. Procédé selon les revendications 1 à 6, dans lequel l'agent de désulfuration est utilisé sous forme réduite à l'étape a).

9. Procédé selon les revendications 1 à 8, dans lequel l'étape a) est réalisée à une température de 40 à 200 °C et à une pression de 1 à 40 bar, de préférence de 1 à 32 bar, notamment de 1,5 à 5 bar.

10. Procédé selon les revendications 1 à 9, dans lequel le catalyseur de ruthénium supporté utilisé à l'étape b) a une teneur en ruthénium de 0,01 à 30 % en poids, par rapport au poids total du catalyseur.

11. Procédé selon les revendications 1 à 10, dans lequel le catalyseur de ruthénium supporté utilisé à l'étape b) contient de l'oxyde d'aluminium en tant que matériau support.

12. Procédé selon les revendications 1 à 10, dans lequel le catalyseur de ruthénium supporté utilisé à l'étape b) contient de l'oxyde de silicium en tant que matériau support.

13. Procédé selon la revendication 12, dans lequel le catalyseur de ruthénium supporté est un catalyseur à enveloppe, au moins 60 % en poids du métal actif se trouvant dans l'enveloppe du catalyseur jusqu'à une profondeur de pénétration de 200 µm.

14. Procédé selon les revendications 1 à 13, dans lequel l'étape b) est réalisée à une température de 50 à 250 °C, de préférence de 60 à 200 °C, notamment de 70 à 170 °C, et à une pression de 1 à 200 bar, particulièrement de 10 à 50 bar, de préférence de 19 à 40 bar, notamment de 25 à 35 bar.

15. Procédé selon les revendications 1 à 14, dans lequel le benzène est utilisé en tant qu'hydrocarbure aromatique.

16. Procédé selon les revendications 1 à 15, dans lequel le produit de réaction obtenu est soumis à une purification supplémentaire lors d'une étape c) supplémentaire.

17. Procédé selon les revendications 1 à 16, dans lequel le produit de réaction obtenu est purifié par distillation lors d'une étape c) supplémentaire.

18. Procédé selon les revendications 1 à 6, 8 à 11 et 14 à 17, dans lequel l'étape a) est réalisée à une pression de 2 à 4,5 bar, à une température de 50 à 180 °C, en présence d'un agent de désulfuration qui contient 35 à 45 % en poids d'oxyde de cuivre, 35 à 45 % en poids d'oxyde de zinc, 10 à 30 % en poids d'oxyde d'aluminium, et qui est utilisé sous forme réduite, et l'étape b) est réalisée à une pression de 19 à 40 bar, à une température de 70 à 170 °C, en présence d'un catalyseur de ruthénium supporté sur de l'oxyde d'aluminium, dont la teneur en ruthénium est de 0,01 à 30 % en poids par rapport au poids total du catalyseur.

19. Procédé selon les revendications 1 à 6, 8 à 10 et 12 à 17, dans lequel l'étape a) est réalisée à une pression de 2 à 4,5 bar, à une température de 50 à 180 °C, en présence d'un agent de désulfuration qui contient 35 à 45 % en poids d'oxyde de cuivre, 35 à 45 % en poids d'oxyde de zinc, 10 à 30 % en poids d'oxyde d'aluminium, et qui est utilisé sous forme réduite, et l'étape b) est réalisée à une pression de 19 à 40 bar, à une température de 70 à 170 °C, en présence d'un catalyseur de ruthénium supporté sur de l'oxyde de silicium, dont la teneur en ruthénium est de 0,01 à 30 % en poids par rapport au poids total du catalyseur.

20. Procédé selon la revendication 18 ou 19, dans lequel l'étape a) est réalisée en présence d'hydrogène.

21. Procédé d'hydrogénation d'hydrocarbures aromatiques selon l'une quelconque des revendications 1 à 20, comprenant également une étape de régénération du catalyseur, qui comprend le rinçage du catalyseur avec un gaz inerte jusqu'à atteindre l'activité initiale ou une partie de l'activité initiale.
